# EUROPEAN PATENT SPECIFICATION

(11) **EP 0 504 064 B1**
(45) Date of publication and mention of the grant of the patent: **13.05.1998**
(21) Application number: 92400669.5
(22) Date of filing: 13.03.1992
(51) Int. Cl.: C07K 5/06, C07K 5/08, A61K 38/55

(54) **Novel thrombin inhibitors**
Neue Thrombin-Inhibitoren
Nouveaux inhibiteurs de la thrombine

(30) Priority: 15.03.1991 EP 91400713
(43) Date of publication of application: 16.09.1992
(73) Proprietor: MERRELL PHARMACEUTICALS INC., Cincinnati, Ohio 45215-6300 (US)
(72) Inventor: Neises, Bernhard, W-7600 Offenburg-Griesheim (DE); Ganzhorn, Axel, F-67380 Lingolsheim (FR); Tarnus, Céline, F-67000 Strasbourg (FR); Broersma, Robert J., Jr., Noblesville, IN 46060 (US)
(74) Representative: Gillard, Marie-Louise

(56) References cited:
- EP-A- 0 410 411
- BIOCHEMISTRY, vol. 24, no. 8, 1985, Washington, DC (US); GELB et al., pp. 1813-1817
- FEBS LETTERS, vol. 220, no. 2, 1987, Amsterdam (NL); SHAM et al., pp. 299-301
- CHEMICAL ABSTRACTS, vol. 107, 1987, Columbus, OH (US); M. STUEBER, p. 758, AN 40336y
- CHEMICAL ABSTRACTS, vol. 110, 1989, Columbus, OH (US); ANGLIKER et al., p. 709, AN 76058
- PEPTIDES, Proceedings of the 12th American Peptide Symposium, 16-21 June 1991, Cambridge, MA (US); R.T. SHUMAN et al., pp. 801-802

## Description

This invention relates to novel polyfluorinated alkyl derivatives of certain tripeptides, to the methods for their preparation, the intermediates therefor , to their use in inhibiting thrombin and lung tryptase and in their end-use application as anti-coagulants useful in treating thrombophlebitis and coronary thrombosis and in the treatment of asthma.
- EP-A₂-0 410 411 relates to analogs of peptidase substrates in which the carboxy terminal carboxy group has been replaced by a pentafluoroethylcarbonyl (-C(O)C₂F₅) group. These peptidase substrate analogs provide specific enzyme inhibitors for a variety of proteases, the inhibition of which exert valuable pharmacological activities and therefore have useful physiological consequences in a variety of disease states.
- A series of highly selective thrombin inhibitors has been disclosed in The Proceedings of the 12th American Peptide Symposium, June 16 - 21, 1991.

The Applicant has now found novel polyfluorinated alkyl derivatives of certain tripeptides.

More specifically this invention relates to the novel compounds of the formulae 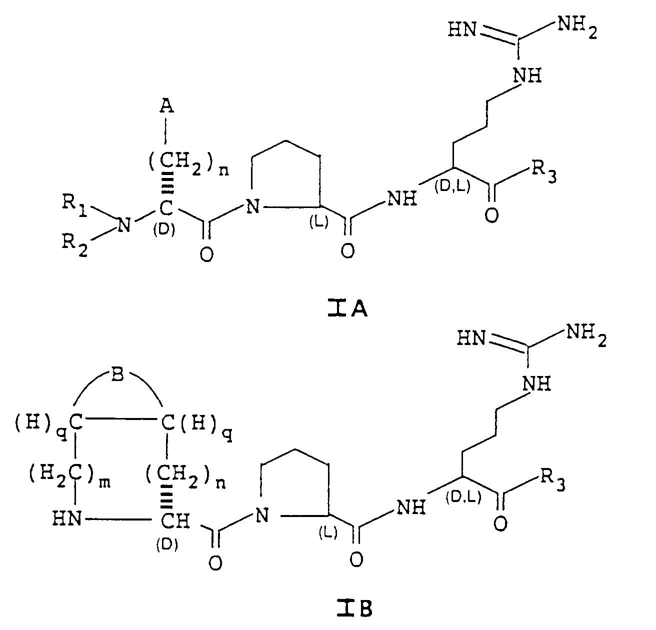 their isomers and mixtures thereof, the hydrates and the pharmaceutically acceptable salts thereof, wherein
- m: is zero, one or two, n is zero or one, with the proviso that the sum of m and n is less than three and greater than zero, q is zero or one with the proviso that the sum of both q's is zero or 2,
- R₁: is H or C₁₋₇ alkyl,
- R₂: is H or C₁₋₇ alkyl, or R₁ and R₂ taken together with the nitrogen atom to which they are attached form a 5- or 6-membered heterocycle,
- R₃: is -CF₂(CH₂)ₜCH₃, -CF₂(CH₂)ₜCOOR₄, -CF₂(CH₂)ₜCONHR₄, -CF₂(CH₂)ₜCH₂OR₄ or -CF₂(CH₂)ᵥCH=CH₂, with t being 2, 3 or 4, and v is 1, 2 or 3,
- R₄: is H or C₁₋₆ alkyl,
- A: is phenyl or cyclohexyl,
- B: is (CH)₄ or (CH₂)₄ which, when taken together with the two carbon atoms to which it is attached, forms a C₆-cyclic hydrocarbon moiety.

The natural amino acids, with the exception of glycine, contain a chiral carbon atom. Unless otherwise specifically indicated, the preferred compounds are the optically active amino acids of the L-configuration except, as indicated, it is preferred that Phe be in its D-configuration.

The tripeptides of formulae IA and IB can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, trifluoroacetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid.

An alkyl group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. In the instance wherein R₁ and R₂ are lower alkyl, it is preferred that one or both be methyl. When R₁ and R₂ form a 5-6-membered heterocycle with the nitrogen atom to which they are attached the preferred moieties are pyrrolidine and piperidine. Preferred compounds are those wherein at least one of R₁ or R₂ is methyl.

In the instance of compounds of Formulae IA and IB, the P₁-α-amino acid residue (arginine) may be in its D- or L-configuration, or a mixture thereof, the P₂-α-amino acid residue (proline) preferably is in its L-configuration, and the α-amino acid residue or substituent on the α-carbon atom of the P₃ moiety (i.e., the 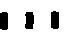 (CH₂)ₙA moiety)
preferably is in its D-configuration, and the preferred residue is Phe and the preferred substituent is a cyclohexylmethyl moiety. In the instance of the compounds of Formula IB, the P₃ moiety is what is called a "TIC" derivative or "TIC-like" derivative (the expression "TIC" being derived from tetrahydroisoquinoline carbonyl). In such instances the bicyclic TIC-moieties formed with the P₃ nitrogen atom and the P₃-α-carbon atom are of the formulae 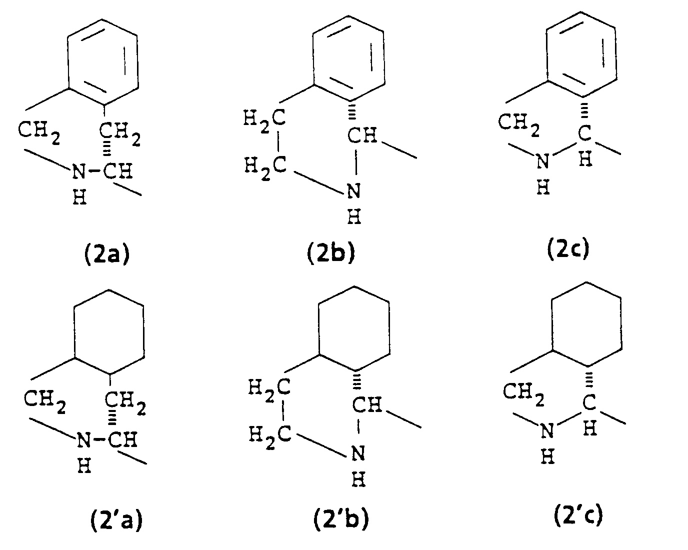 wherein (2a) and (2b) represent a 1,2,3,4-tetrahydroisoquinolinyl moiety, (2'a) and (2'b) represent a 1,2,3,4-decahydroisoquinolinyl moiety, and (2c) and (2'c) represent 2,3-dihydro-1H-isoindolyl and octahydro-1H-isoindolyl moieties, respectively. For convenience the moieties hereinafter may also be referred to as TIC-like modifications.

The compounds of this invention may be prepared by procedures analogously known in the art. In essence the synthesis of the fluorinated alkyl tripeptides of Formula I relies on a modified Dakin-West reaction of 2-phenyl-5(4H)-oxazolone and the anhydrides or acyl halides of trifluoroacetic acid, pentafluoropropionic acid or difluoropentenoic acid, (depending on the desired R₃ moiety), to yield the requisite polyfluoro alkyl ketone amino acid derivatives for use as key intermediates. Further reactions then allow for the conversion of these amino acid analogs to the desired peptides of Formula I. These reactions are illustrated in the following reaction schemes: 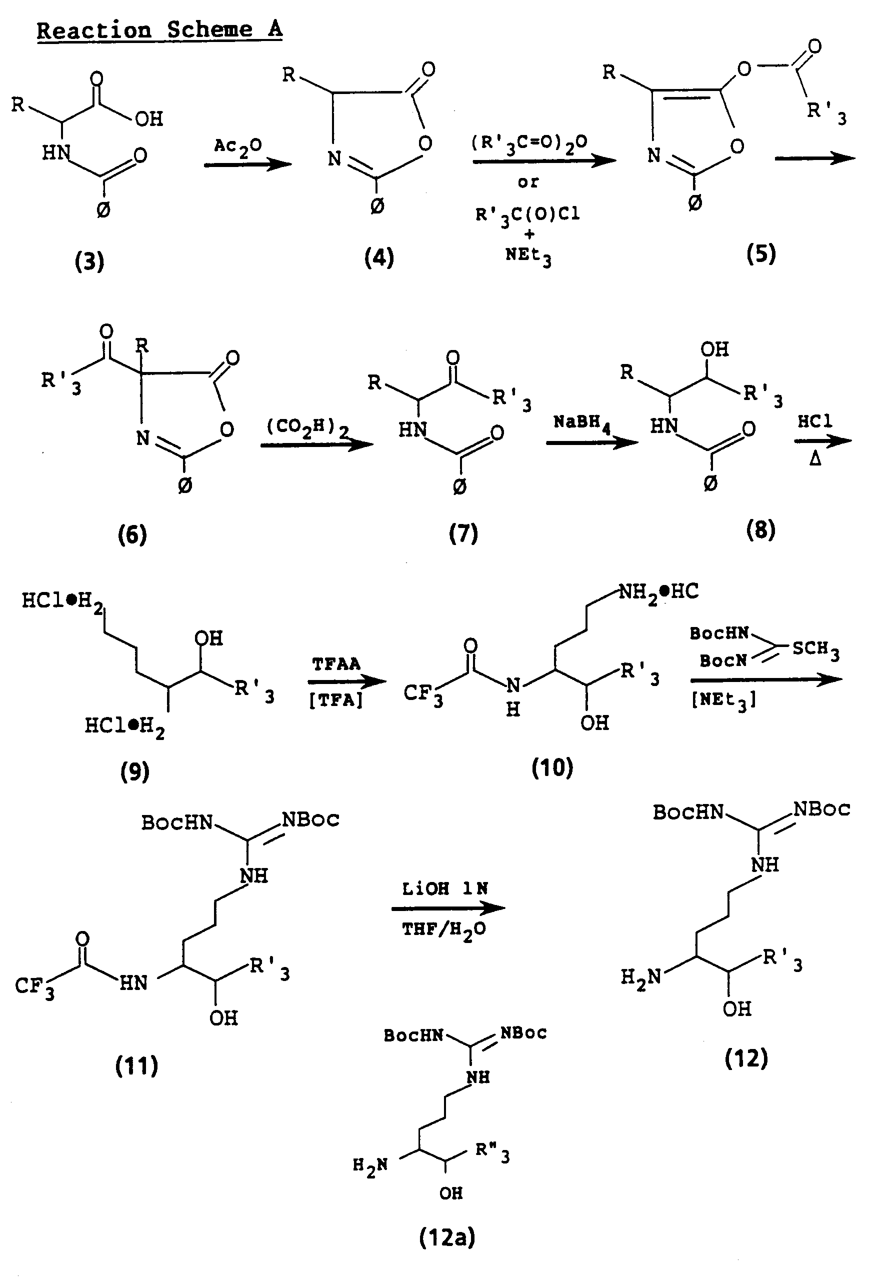 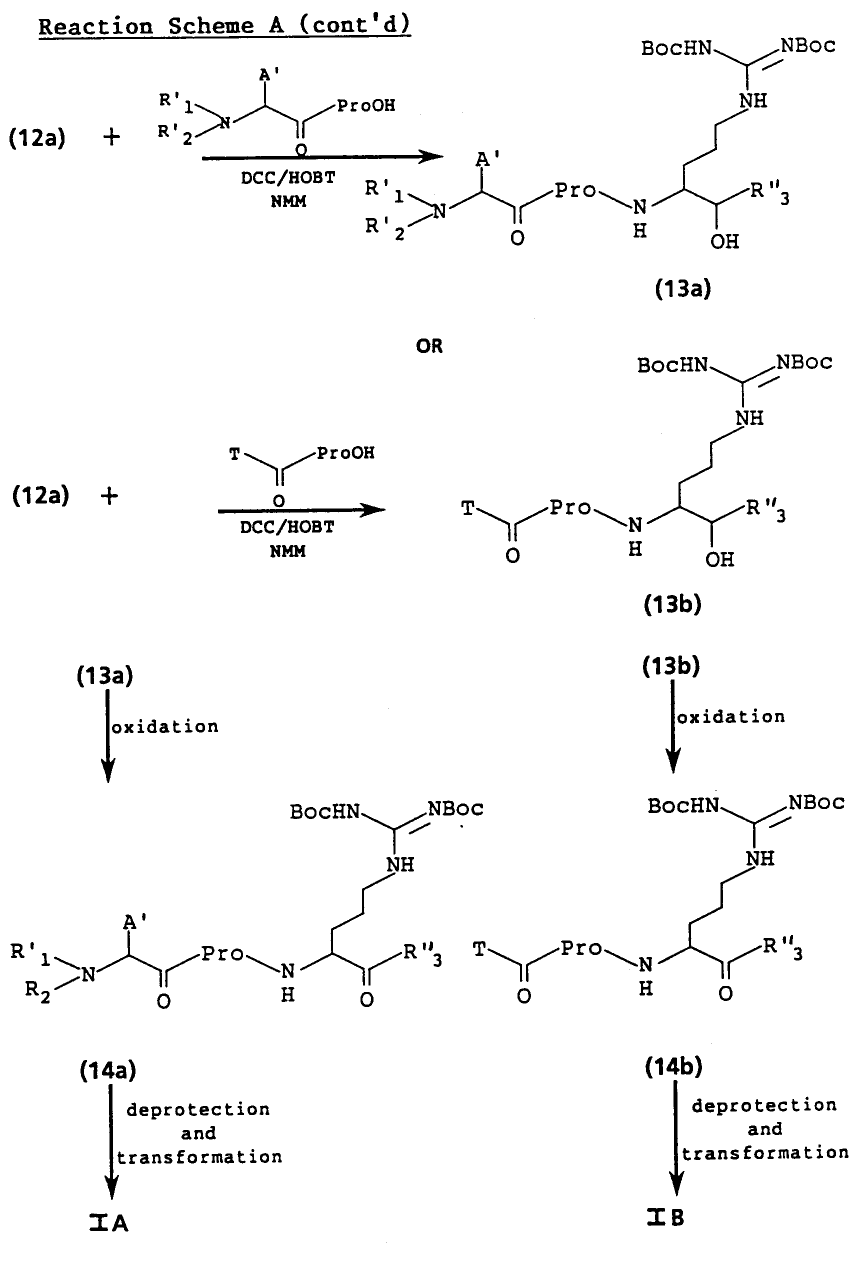 wherein Ø is phenyl, A' is 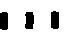 (CH₂)ₙA, n is zero or one,
- R: is ØC(O)NH(CH₂)₃,
- R'₁: is an N-protecting group,
- R'₂: is H or C₁₋₇ alkyl,
- R'₃: is -CF₂(CH₂)ₜC(O)NHR₄, -CF₂(CH₂)ₜCH₂OR₄, or -CF₂(CH₂)ᵥCH=CH₂,
- R''₃: is -CF₂(CH₂)ₜC(O)NHR₄, -CF₂(CH₂)ₜCH₂OR₄, -CF₂(CH₂)ᵥCH=CH₂ or -CF₂(CH₂)ₜCOOR₄,
- T: is a TIC-like moiety as defined above and depicted as 2a, b and c and 2'a, b and c,
- DCC: is dicyclohexylcarbodiimide,
- NMM: is N-methylmorpholine,
- HOBt: is hydroxybenzotriazole (hydrate),
- TFA: is trifluoroacetic acid,
- TFAA: is trifluoroacetic acid anhydride, and A, R₄, t, v, IA and IB are as previously defined. The preferred amine protecting groups are Boc or CBz and, although the preferred protecting group on the arginine moiety is the depicted Boc group, CBz may also be utilized.

In effecting the foregoing reactions, the N-bis-benzoylated amino acid (3) is cyclized by standard techniques to the 5(4H)-oxazolone (4) (Ac₂O, 30 minutes, 90°C oil bath temperature) in good yield. Evaporation of the solvents affords a highly pure compound which can be used without further purification for the next step. Reaction of the oxazolone (4) using a modified Dakin-West procedure, with the appropriate polyfluoroacid acyl halide (with NEt₃) or anhydride, is accomplished at 40°C (oil bath temperature) under an atmosphere of N₂ for 24 hours (¹H- and ¹⁹F-NMR-monitoring). When all of the starting material (4) is consumed, the C-4-acylated oxazolones (5) are the main products. Residual anhydride and polyfluorinated acids formed are removed under vacuum [1.333 Pa (0.01 torr); 25-70°C oil bath temperature, acetone/dry ice trap]. Alternatively, compounds (3) may be directly converted to (6) by treatment with the R'₃ anhydrides or R'₃ acyl halides, and in such instances the intermediates are not isolated. The oily residues are then mixed with a saturated solution of freshly prepared anhydrous oxalic acid in tetrahydrofuran. Commercial oxalic acid is dried for 16 hours at 100°C in a drying oven. Two subsequent sublimations [1.333 Pa (0.01 Torr), 90°C] afford anhydrous oxalic acid (m.p. 104°C) which is transferred under an atmosphere of N₂ into a flask and stoppered with a septum. Anhydrous tetrahydrofuran is added to the solid until most of it has dissolved (about 4 ml/g) and the resulting solutions are stirred at room temperature for about 16 hours, when gas evolution has completely ceased. Work-up (e.g., EtOAc/H₂O, aqueous NaHCO₃, brine; drying over MgSO₄) gives in satisfactory yield the desired fluorinated compounds (7) as mixtures of the ketones and their hydrated forms.

For the intended transformation of the α-benzamides (7) the polyfluoroalkyl ketone functionality has to be temporarily masked. This can be achieved by reduction of the ketones (7) (NaBH₄;EtOH) to the alcohols (8). The two benzamido functions are cleaved by acid hydrolysis to produce the diaminopolyfluoroalkyl alcohols (9). Regioselective protection of the lateral amino group of the diamino alcohols (9) as a trifluoroacetamide (10) is effected with trifluoroacetic anhydride in trifluoroacetic acid. Guanylation of the ω-amino group into the fully protected arginine analogs (11) is effected with bis-Boc-S-methylisothiourea in triethylamine. Liberation of the lateral-amino protecting function is effected with lithium hydroxide in tetrahydrofuran/water to yield the compounds of Formula (12) which are ready for coupling with the appropriate reactants (i.e.,R'₁,R'₂N-CH(A')C(O)ProOH and the TICC(O)ProOH) according to standard procedures well known in the art [Nicolaides, E., DeWald, H., Westland, R., Lipnik, M., and Posler, J., *J. Med. Chem.* 11, 74 (1968)] to give the completely protected tripeptide alcohol analogs of arginine (13a) and (13b), respectively, which are ready for oxidation of the alcohol function to the corresponding ketones. It is to be noted that compounds (12a) differ in scope from compounds (2) in that it adds the moiety -CF₂(CH₂)ₜCOOR₄ to the R'₃ moiety. Thus, R''₃ embraces the R'₃ and the -CF₂(CH₂)ₜCOOR₄ moieties; the latter being prepared according to Reaction Scheme B.

Although there are several procedures available for the required oxidation, the most preferred method for conversion of (13a) and (13b) to their corresponding ketones (14a) and (14b), is the Swern oxidation procedure. In general the Swern oxidation [see Synthesis, (1981), 165] is effected by reacting about 2 to 10 equivalents of dimethylsulfoxide (DMSO) with about 1 to 5 equivalents of trifluoroacetic anhydride [(CF₃CO)₂O] or oxalyl chloride [(COCl)₂], said reactants being dissolved in an inert solvent, e.g., methylene chloride (CH₂Cl₂), said reaction being under an inert atmosphere (e.g., nitrogen or equivalently functioning gas) under anhydrous conditions at temperatures of about -70°C to -30°C to form an *in situ* sulfonium adduct to which is added about 1 equivalent of the appropriate alcohols, i.e., compounds (13a) or (13b). Preferably, the alcohols are dissolved in an inert solvent, e.g., CH₂Cl₂ or minimum amounts of DMSO, and the reaction mixture is allowed to warm to about -20°C (for about 10-20 minutes) and then the reaction is completed by adding about 3 to 10 equivalents of a tertiary amine, e.g., triethylamine, N-methyl morpholine, etc.

Another alternative process for converting the alcohols to the desired ketones is an oxidation reaction which employs periodinane (i.e., 1,1,1-triacetoxy-2,1-benzoxiodol-3-(3H)-one), [see Dess Martin, *J. Org. Chem.*, **48**, 4155, (1983)]. This oxidation is effected by contacting about 1 equivalent of the alcohols with 1 to 5 equivalents of periodinane (preferably 1.5 equivalents), said reagent being in suspension in an inert solvent (e.g., methylene chloride) under an inert atmosphere (preferably nitrogen) under anhydrous conditions at 0°C to 50°C (preferably room temperature) and allowing the reactants to interact for about 1 to 48 hours. Deprotection of the amine protecting groups may be effected as desired after the ketones have been isolated.

Alternatively, a modified Jones oxidation procedure may conveniently be effected by reacting the alcohols with pyridinium dichromate by contacting the reactants together in a water-trapping molecular sieve powder, e.g., a grounded 3 Angström molecular sieve), wherein said contact is in the presence of glacial acetic acid at about 0°C to 50°C, preferably at room temperature followed by isolation and then removing amine protecting groups.

Alternatively, 1 to 5 equivalents of a chromic anhydride-pyridine complex may be used (i.e., a Sarett reagent prepared *in situ*) [see Fieser and Fieser "Reagents for Organic synthesis" Vol. 1, pp. 145 and Sarett, et al., *J.A.C.S.* 25, 422, (1953)] said complex being prepared *in situ* in an inert solvent (e.g., CH₂Cl₂) in an inert atmosphere under anhydrous conditions at 0°C to 50°C to which complex is added 1 equivalent of the alcohols allowing the reactants to interact for about 1 to 15 hours, followed by isolation and removing amine protecting groups.

In the special instance wherein R₃ is -CF₂(CH₂)ₜCOOR₄, the procedure for preparing necessary intermediates (corresponding to compounds of Formula (12), but wherein the desired R₃ is -CF₂(CH₂)ₜCOOR₄), the reactions of the following reaction scheme may be utilized: 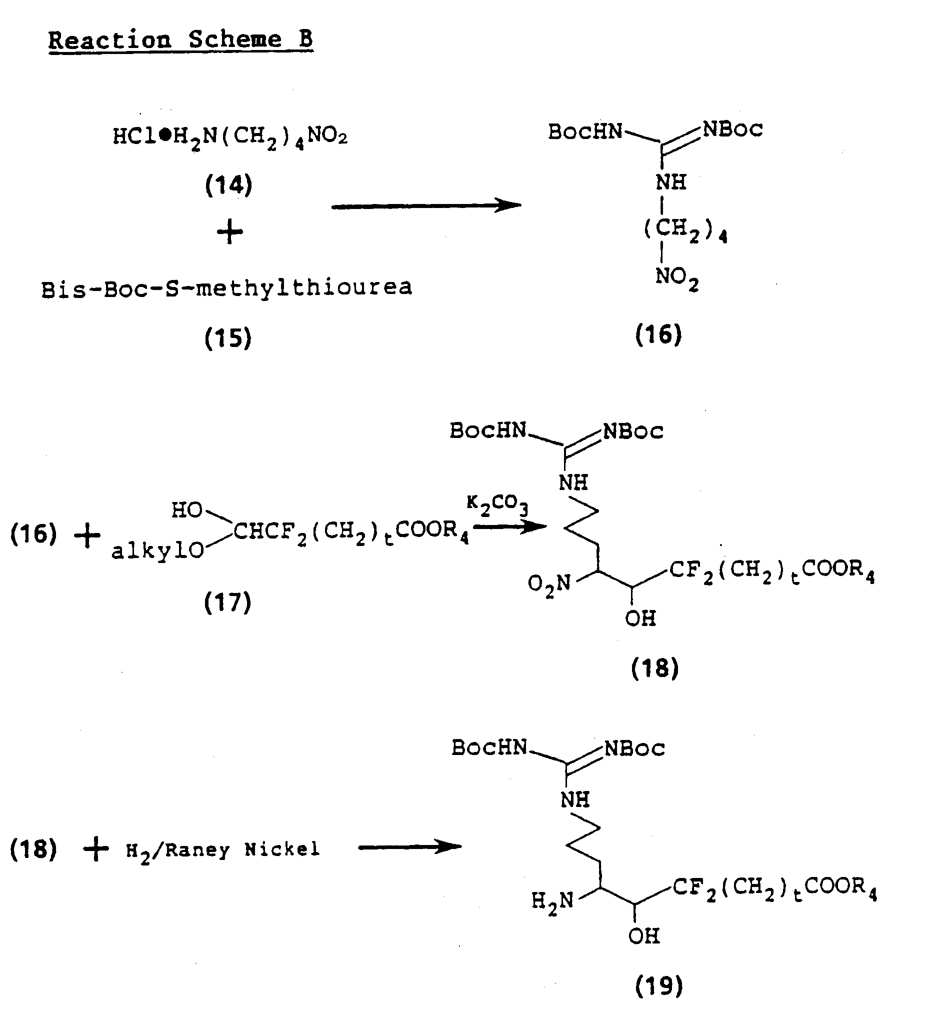 wherein Boc, R₄ and t are as previously defined.

The reactants (17) may be prepared by reacting bromodifluoroethyl acetate [see R.H. Abeles and Ch. P. Govardham, *Archives of Biochemistry and Biophysics*, **280**, 137 (1990)] with the appropriate aldehyde, under Reformatsky conditions, to the corresponding difluoro diester alcohols. These alcohols are then mesylated, the mesylated products subjected to elimination by reaction with diazabicycloundecane (DBU) and the so-obtained olefins are reduced by hydrogenation; said mesylation, elimination and hydrogenation being effected by standard and well-known procedures and techniques such as are described in Hing L. Sham et al., *Biochem. and Biophys. Res. Comm.*, **175**, 914 (1991). The so-produced intermediates (19), which are incorporated into the structural formula (12a), are then processed as described in Reaction Scheme A.

In the special instance wherein it is desired to prepare compounds wherein R₃ is -CF₂(CH₂)₂CH₃, the acid anhydride or acyl halide reactants used to prepare compounds of Formula (5) would bear the moiety -CF₂CH₂CH=CH₂ and, when compounds (14a) and (14b) are subjected to transformations, the double bond of the olefinic (e.g., -CF₂CH₂CH=CH₂) moiety may also be reduced. In the special instance wherein R₃ is -CF₂(CH₂)ₜCH₃ with t being 3 or 4, the corresponding olefins (e.g., -CF₂CH₂CH=CH-CH₃ or -CF₂CH₂CH=CHCH₂CH₃) would be reduced. For the preparation of these latter type olefins, the starting acyl halides or anhydrides can be prepared by the methods described by R. W. Lang et al., *Tetrahedron Letters,* **29**, 3291 (1988).

Further processing of the oxidized intermediates [(14a) and (14b)] to the desired compounds of Formulae IA and IB depends essentially on the definition of the R₁, R₂ and R''₃ moieties. In those instances wherein it is desired to prepare final compounds wherein R₁ is H and R₂ is an alkyl, then the intermediates to be utilized would be those wherein R'₁ is a protecting group and R'₂ is a C₁₋₇ alkyl. In such instances the R'₁ protecting group and the two protecting groups on the arginine moiety would be removed by techniques well known in the art, such as by reaction with HCl gas/ether or TFA/CH₂Cl₂. In those instances wherein both R₁ and R₂ are H, then R'₁ would be a protecting group and R'₂ would be H and again all three protecting groups (preferably Boc) would be removed as above. In those instances wherein both R₁ and R₂ are other than H, then R'₁ is a protecting group (preferably CBz) and R'₂ is H and such intermediates (14a) would be reacted with an appropriate aldehyde (e.g., formaldehyde, glutaraldehyde or succinaldehyde) in the presence of H₂/Pd(OH)₂/carbon in isopropanol to form the desired N,N-dialkyled pyrrolidine or piperidine derivatives. The remaining two Boc-protecting groups on the arginine moiety would be cleaved by treatment with HCl/Et₂O or TFA in CH₂Cl₂ according to standard techniques well known in the art. In the instance wherein it is desired to convert the olefinic moiety (e.g., a -CF₂CH₂CH=CH₂ moiety) to its saturated analog (e.g., a -CF₂(CH₂)₂CH₃ moiety), the conversion is effected by standard hydrogenation techniques, preferably concurrently with the removal of the amino-protecting groups.

The following Example 2 illustrates the preparation of the compounds of Formula I. Example 1 does not concern any of the compounds of the invention as presently claimed.

### EXAMPLE 1

### L-Prolinamide, N-methyl-D-phenylalanyl-N-[1-[3-[(aminoiminomethyl)amino]propyl]-3,3,4,4,4-pentafluoro-2-oxobutyl]-, dihydrochloride, hydrate

### STEP A:

### N,N'-[1-(Pentafluoropropionyl)-1,4-butanediyl]-bis(benzamide), hydrate

Pentafluoropropionic anhydride (31.8 ml, 50 g, 161 mmol) was added under an atmosphere of N₂ to a well stirred powder of the ornithine 5(4H)-oxazolone (see Example 1, Step 2; Reaction Scheme A, Structure (2); 14.05 g, 4.3 mmol). The resulting mixture was stirred at 40°C for 16 hours. At this time an aliquot (about 50 mg) was taken for a ¹H- and ¹⁹F-NMR spectrum (CDCl₃). The disappearance of the ¹H-signal at 4.5 ppm indicates disappearance of oxazolone, whereas the ¹⁹F-signals at 42.7; 46.7 (2s, 2 × CF₂) and 80.0; 80.5 (2s, 2 x CF₃) indicate the formation of 3'-N-4-bispentafluoropropionyl oxazolone. The additional ¹⁹F-signals were assigned to the excess of pentafluoropropionic anhydride (PFPAA) and the pentafluoropropionic acid (PFPA) formed. Solvents were then evaporated at 55-60°C [66.65-133.3 Pa (0.5-1 Torr, dry ice-acetone trap)] for about 6 hours to give a thick orange oil. A ¹⁹F-NMR spectrum of another aliquot indicates disappearance of all PFPAA and PFPA. At this time 40 ml of a saturated solution of oxalic acid (15.0 g, 150 mmol) in tetrahydrofuran was added and the resulting orange oil was stirred at 55°C for 6 hours when effervescence has totally stopped. The solvent was evaporated [2.666.10³ Pa (20 Torr, 30°C)] and the oily residue dissolved in AcOEt. This solution was stirred for 15 minutes at room temperature with a saturated solution of KHCO₃ (intended hydrolysis of pentafluoropropionamide). Phases were separated and the organic layer was washed with water, 1N HCl, and brine, dried over MgSO₄ and evaporated [2.666 10³ Pa (20 Torr, 30°C); then 13.33 Pa (0.1 Torr, 30°C)]. The resulting orange oil (21.7 g) was subjected to flash chromatography on silica gel (500 g; eluent AcOEt/petroleum ether: 1/3) in two batches of about 11 g. Fractions 11-20, containing the N-pentafluoropropionamide **A-2** were evaporated to give 7.0 g (27%). Fractions 28-105 containing the ketone **A-1** were evaporated to afford 10.1 g (52%) as a white solid. Total yield: 79% based on oxazolone.
- **A-1**: ¹H-NMR (CDCl₃) δ = 8.0-7.85 (m, 5H, aryl, NH); 7.8-7.4 (m, 6H, aryl), 5.5-5.3 (m, 1H, CHCO), 3.9 (broad, t, 2H, NCH₂), 2.3-1.8 (m, 4H, 2CH₂).
¹⁹F-NMR (CDCl₃) δ = 40.33 (d, J = 7.5 Hz, CF₂CO), 46.67 (s, CF₂CONH), 80.0 (s, 2CF₃).
- **A-2**: ¹H-NMR (CDCl₃) δ = 8.1-7.8 (m, 4H, aryl), 7.7-7.4 (m, 7H, aryl,NH), 6.6 (m, 1H, NH), 5.3 (m, 1H, CHCO), 3.7 (broad t, 2H, NCH₂), 2.4-1.8 (m, 4H, 2CH₂).
¹⁹F-NMR (CDCl₃/C₆F₆) δ = 40.3 (d, J= 7.5 Hz, CF₂), 80.0 (s, CF₃).
- MS: (CI/NH₃): 443 (MH⁺).

A small sample of **A-2** (100 mg) was allowed to crystallize from AcOEt/petroleum ether to give 80 mg of analytically pure title compound:

| Analysis calculated for C₂₁H₁₉O₃N₂F₅ (442.39): | | | |
|---|---|---|---|
| | C: 57.02; | H: 4.33; | N: 6.33 |
| Found: | C: 57.14; | H: 4.23; | N: 6.36. |

### STEP B:

### N,N'-[1-(2,2,3,3,3-Pentafluoro-1-hydroxypropyl)-1,4-butanediyl]-bis(benzamide)

The reduction of the two ketones **A-1** and **A-2** was performed in two separate reactions:

### 1) Reduction of A-1:

NaBH₄ (0.43 g, 11 mmol) was added in one portion to a cooled (0°C) and stirred solution of the pentafluoroethyl ketone **A-1** (10.1 g, 17.1 mmol) in EtOH (130 ml). The mixture was allowed to warm up to room temperature and further stirred for one hour. Hydrochloric acid (6N) was added carefully until effervescence has stopped. The solution was neutralized with Na₂CO₃ and EtOH evaporated. The resulting mixture was redissolved in AcOEt/water and phases separated. The aqueous layer was extracted twice with AcOEt and the combined organic phases washed with water and brine. Drying over MgSO₄ and evaporation of solvents afford a white solid, which was subjected to flash chromatography on silica gel (300 g, eluent AcOEt/petroleum ether: 1/1, then 4/1). Product-containing fractions were evaporated to give 5.88 g (77%) of the desired alcohol as a white solid. Rf = 0.45-0.50 (AcOEt/petroleum ether: 1/1), two badly separated spots for the diastereoisomers.

### 2) Reduction of A-2:

As described above for **A-1**: 6.91 g (15.6 mmol) of the ketone **A-2**, 300 mg (7.9 mmol) NaBH₄, and 90 ml of EtOH afforded 6.05 g (89%) of the alcohol N,N'-[1-(2,2,3,3,3-pentafluoro-1-hydroxypropyl)-1,4-butanediyl]-bis(benzamide) as a white solid, which was in all aspects comparable to the compound obtained above.
¹H-NMR (CDCl₃,CD₃OD) δ = 7.8-7.5 (m, 4H, aryl), 7.45-7.1 (m, 6H, aryl), 4.5 and 4.2 (2m, 1H, CHOH, ratio 3:1), 3.5 (m, 4H, 2CH₂).
¹⁹F-NMR (CDCl₃, CD₃OD, C₆F₆) δ = ABX system centered at 36.3; A: 40.3 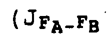 = 280 Hz, 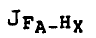 = 3 Hz); B: 32.3 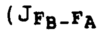 = 280 Hz, 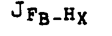 = 30 Hz, CF₂), 79.0 (s, CF₃) = diastereoisomer 1.
ABX system centered at 35.3; A: 36.3, B: 33.3 (with equal coupling constants as mentioned above) = diastereoisomer 2; ratio 3/1.

| Analysis calculated for C₂₁H₂₁O₃N₂F₅; (444.40) | | | |
|---|---|---|---|
| | C: 56.76; | H: 4.76; | N: 6.30 |
| Found: | C: 56.94; | H: 4.83; | N: 6.29. |

### STEP C:

### 4,7-Diamino-1,1,1,2,2-pentafluoro-3-heptanol, dihydrochloride

A stirred solution of the above-prepared alcohol (Step B) (11.78 g, 26.6 mmol) in concentrated aqueous hydrochloric acid (240 ml) was heated under stirring to reflux while the progress of hydrolysis was followed by TLC (BuOH/water/AcOH = 4/1/1). After 16 hours of reaction time solvent was evaporated and the oily residue subjected a second time to the above conditions. When the complete formation of the bisamino alcohol was indicated by TLC, the solution was cooled to room temperature and solvents evaporated. The oily residue was dissolved in water and the solution washed with Et₂O (3 × 100 ml). The aqueous layer was evaporated to dryness to afford 8.14 g (99%) of the desired diamino alcohol as a brownish foam.
¹H NMR: (D₂O) δ = 4.6 (m, 1H, CHOHCF₂), 3.1 (m, 2H, NCH₂), 3.6 (m, 1H, CHN), 2.0 (m, 4H, 2CH₂).

### STEP D:

### 4-Trifluoroacetylamino-7-amino-1,1,1,2,2-pentafluoro-3-heptanol, hydrochloride

Trifluoroacetic anhydride (3.55 ml, 25 mmol) was added dropwise to a stirred solution of the diamino alcohol of Step C (3.09 g, 10 mmol) in 50 ml trifluoroacetic acid. After two hours of stirring at room temperature, another 2.5 ml of TFAA was added to the solution and stirring was continued for 10 hours. The solution was evaporated to dryness, giving a brown oil. Trituration with Et₂O/petroleum ether afforded a brownish solid, which was filtered and washed with petroleum ether. Drying gave a slightly colored solid of the title compound (3.48 g, 95%), which was pure enough for the following reaction.
¹H-NMR (D₂O) δ = 4.6 (m, 2H, 2CH), 3.1 (m, 2H, NCH₂), 2.0-1.7 (m, 4H, 2CH₂).
¹⁹F-NMR (D₂O, ref. CF₃CO₂H) δ = ABX system centered at -49.00) A: -44.00 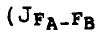 = 280 Hz); B: -54.00 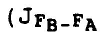 = 280 Hz) 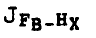 = 30 Hz) = isomer 1; ABX centered at -49.33, A: 45.00, B: 40.67 (coupling constants as above) = isomer 2; ratio 4:1.
MS: (CI/NH₃): 333 (MH⁺).

### STEP E:

### N-[1-[7-bis[(1,1-Dimethylethoxy)carbonyl]-amino]methylene]amino]-N(4-trifluoroacetylamino)-3-hydroxy-1,1,1,2,2-pentafluoroheptane

Bis-Boc-S-methylisothiourea (7,3 g, 25 mmol) was added under an atmosphere of N₂ to a well stirred solution of the hydrochloride salt of Step D (5.1 g, 10 mmol) and NEt₃ (3.5 ml, 25 mmol) in anhydrous tetrahydrofuran (100 ml). The mixture was stirred at 40°C for 60 hours. Solvents were evaporated (a trap filled with an aqueous solution of KMnO₄/Na₂CO₃ was placed between the flask and the pump to avoid poisoning with methanethiol), and the oily residue dissolved in AcOEt. This solution was washed with water, saturated solutions of citric acid, NaHCO₃, and brine. Drying over MgSO₄ and evaporation of the solvents afforded an oil (10 g) which was subjected to flash chromatography on silica gel (50 g, eluent AcOEt/petroleum ether: 1/8, then 3/1). Product-containing fractions were evaporated to afford 2.92 g (51%) of the protected ω-guanidino-γ-amino alcohol as a colorless foam.
¹H-NMR (CDCl₃, 360 MHz) δ = 11.20 (s, 1H, NH), 10.31 (d, J = 10 Hz, 1H, NHCOCF₃), 9.73 (broad s, 1H, NHCH₂), 4.45 (t, J = 9.5 Hz, 1H, CHN), 4.25 d, J = (22 Hz, CHOH), 3.75 (m, 1H, OH), 3.65 and 3.23 (2m, 2H, NCH₂), 2.1 and 1.9 (2m, 4H, 2CH₂), 1.45 and 1.40 (2s, 18H, 2 tert-Boc).
¹⁹F-NMR (CDCl₃, ref. C₆F₆) δ = ABX system centered at 35.50; A: 39.33 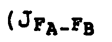 = 277 Hz), B: 32.30 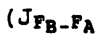 = 277 Hz; 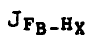 = 22 Hz) = isomer 1; ABX system centered at 34.00; A: 40.05 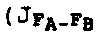 = 277 Hz), B: 27.5 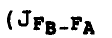 = 277 Hz, 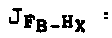 = 22 Hz) = isomer 2, ratio 4:1; 78.87 and 79.40 (2s, ratio 4:1, CF₃); 86.12 and 86.53 (2s, ratio 1:4, CF₃CO).

| Analysis calculated for C₂₀H₃₀F₈N₄O₆.0.5 H₂O (574.47): | | | |
|---|---|---|---|
| | C: 41.17; | H: 5.36; | N: 9.60. |
| Found: | C: 41.06; | H: 5.15; | N: 9.57. |

### STEP F:

### N-[1-[7-[bis(1,1-Dimethylethoxy)carbonyl]amino]methylene]amino-4-amino-3-hydroxy-1,1,1,2,2-pentafluoroheptane

A freshly prepared aqueous solution of LiOH (1N, 7 ml) was added to a stirred solution of the trifluoroacetamide of Step E (3.0 g, 5.2 mmol) in tetrahydrofuran/water (9/1, 50 ml). The solution was stirred at room temperature for 20 hours, when starting material has disappeared (TLC, AcOEt/petroleum ether: 1/5). Tetrahydrofuran was evaporated and the aqueous solution extracted with Et₂O (4 × 50 ml). The combined extracts were washed with water and brine, and dried over MgSO₄. Evaporation of the solvent afforded 2.18 g (88%) of the above-described aminoalcohol as a white solid.
¹H-NMR (CDCl₃) δ = 11.5 (m, 1H, NH), 8.4 (m, 1H, NH), 4.1 and 3.9 (2m, 1H, CHOH), 3.5 and 3.2 (2m, 3H, CHN, NCH₂), 2.5 (m, 2H, NH₂), 1.9-1.4 (m, 4H, 2CH₂), 1.50 (s, 18H, 2 tert-Boc).
¹⁹F-NMR (CDCl₃) δ = ABX system centered at 37.3; A: 43.67, 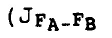 = 280 Hz; 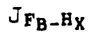 = 22 Hz, CF₂, isomer 1), 79.0 (s, CF₃) and ABX system centered at 39.0 (coupling constant as above), (CF₂ isomer 2), 79.5 (s, CF₃); ratio 4:1.

| Analysis calculated for C₁₈H₃₁F₅N₄O₅ (478.46): | | | |
|---|---|---|---|
| | C: 45.19; | H: 6.53; | N: 11.71. |
| Found: | C: 45.13; | H: 6.44; | N: 11.56. |

### STEP G:

### L-Prolinamide, N-[(1,1-dimethylethoxy)carbonyl]-N-methyl-D-phenylalanyl-N-[1-[3-[[bis[[(1,1-dimethylethoxy)carbonyl]amino]methylene]amino]propyl]-3,3,4,4,4-pentafluoro-2-hydroxybutyl]-

Dicyclohexylcarbodiimide (0.96 g, 4.7 mmol) was added to a stirred and cooled (0°C) solution of Boc-N(methyl)-D-Phe-Pro-OH (1.78 g, 4.7 mmol) and N-hydroxybenzotriazole (0.711 g, 4.7 mmol) in CH₂Cl₂ (50 ml). The mixture was stirred for 30 minutes at 0°C, when the above-prepared amino alcohol (Step F) (2.22 g, 4.64 mmol) and NMM (0.52 ml, 4.7 mmol) were added. The resulting mixture was further stirred for 0.5 hour at 0°C and then allowed to warm up to room temperature. Stirring was continued for 16 hours at room temperature. Filtration of the precipitated DCU and washing of the filtrate with saturated solutions of citric acid, KHCO₃, and brine was followed by drying over MgSO₄ and evaporation of the solvent to afford a viscous oil. Flash chromatography on silica gel (150 g, eluent AcOEt/petroleum ether: 1/1) and evaporation of the product-containing fractions afforded 3.09 g (70%) of the desired tripeptide alcohol as a colorless oil.
¹H-NMR: (CD₃OD, 360 MHz) δ = 7.2 (m, 5H, aryl), 5.1-4.9 (2m, 1H, CH-Phe), 4.4-3.9 (m, 3H, CH-Pro, CHOH, CHN), 3.7-3.3 and 3.2-2.9 (2m, 6H, 2NCH₂, Phe-CH₂), 2.85-2.7 (5s, 3H, NCH₃), 2.3-1.6 (m, 8H, 4CH₂), 1.5-1.1 (6s, 27H, 3 tert-Boc).
¹⁹F-NMR (CD₃OD, C₆F₆ ext. ref., 360 MHz) δ = about 3 ABX systems centered at 43.7 (CF₂ - isomer 1 and 2; cis-trans-isomers) and 81.80, 81.55, 81.30 (3s, CF₃ of diff. isomers).
MS (FAB): 837 (MH⁺).

### STEP H:

### L-Prolinamide, N-[(1,1-dimethylethoxy)carbonyl]-N-methyl-D-phenylalanyl-N-[1-[3-[[bis[[(1,1-dimethylethoxy)carbonyl]amino]methylene]amino]propyl]-3,3,4,4,4-pentafluoro-2-oxo-butyl]-

A 100 ml three-necked flask equipped with a magnetic stirring bar, thermometer, and a N₂-inlet was charged with a solution of oxalylchloride (0.52 ml, 5.7 mmol) in 5 ml of anhydrous CH₂Cl₂. After cooling the solution to -60°C, a solution of dimethylsulfoxide (1.2 ml, 14.3 mmol) in 10 ml of anhydrous CH₂Cl₂ was added via a syringe at a rate to keep internal temperature at -55°C. The mixture was stirred for 15 minutes at -55°C, when the alcohol of Step F (3.0 g, 3.58 mmol) in 20 ml of anhydrous CH₂Cl₂ was added dropwise. After complete addition, the cooling bath was removed and stirring continued until internal temperature reaches -20°C. At this temperature stirring was continued for about 5 minutes and the solution cooled again to -55°C. At this temperature NEt₃ (2.5 ml, 17.9 mmol) was added at a rate to keep internal temperature at -55°C. Finally a saturated citric acid solution (10 ml) was added. The mixture was allowed to warm up to room temperature and 200 ml of CH₂Cl₂ was added. Phases were separated and the organic layer washed with water, a saturated solution of NaHCO₃, and brine. Drying over MgSO₄ and evaporation of solvents give a colorless oil (about 3 g) which was subjected to flash chromatography on silica gel (100 g, eluent AcOEt/petroleum ether: 1/2, then 1/1, then 2/1). Product-containing fractions were evaporated to obtain 1.0 g (33%) of the above-described pure pentafluoro ketone as a colorless foam. About 1.6 g of a mixture of the above-described ketone and starting alcohol described in Step G was recovered, which can be recycled for another oxydation.
¹H-NMR (CDCl₃, 360 MHz) δ = 11.5 (m, 1H, NH), 8.5 (m, 1H, NH), 7.8 (m, 1H, NH), 7.2 (m, 5H, aryl), 5.1-4.3 (m, 3H, α-CH-Phe, α-CH-Pro, α-CHCO), 3.7-3.1 and 3.1-2.6 (2m, 9H, 2NCH₂, CH₂C₆H₅, NCH₃), 2.2-1.6- (m, 8H, 4CH₂), 1.5-1.2 (m, 27H, 9CH₃).
¹⁹F-NMR (CDCl₃, 360 MHz) δ = 40.33 and 40.19 (2s, CF₂CO), ABX systems centered at 39.0 (CF₂), 80.0 (s, CF₃), 82.7 and 82.9 (2s, CF₃), ratio 4:1.

### STEP I:

### L-Prolinamide, N-methyl-D-phenylalanyl-N-[1-[3-[(aminoiminomethyl)amino]propyl]-3,3,4,4,4-pentafluoro-2-oxobutyl]-, dihydrochloride, hydrate

0.9 g (1.07 mmol) of the tripeptide derivative of Step H was dissolved in 50 ml on anhydrous Et₂O. 200 ml of a saturated HClgas/Et₂O solution was added and the resulting solution stirred at room temperature for 48 hours (under exclusion of moisture). Petroleum ether (about 100 ml) was added and the precipitate filtered under N₂. Drying of the filter residue (0.1 Torr, 40°C) afforded 0.6 g of a white amorphous powder which was dissolved in water (20 ml) and the solution filtered over a Millipore® filter disk. The filtrate was then lyophilized to give 0.5 g (82%) of the title compound as a white fluffy powder.
¹H-NMR (D₂O) δ = 7.55 (m, 3H, aryl), 7.30 (m, 2, aryl), 4.55 (m, 1H, CH-Phe), 4.38 (m, 1H, CH-Pro), 4.27 [m, 1H, CHN-C(OH)₂], 3.50 (m, 1H, HCH_{A}-Pro), 3.37 (m, 1H, C₆H₅CH_{A}), 3.25 (m, 2H, NCH₂-guanidine), 3.15 (m, 1H, C₆H₅CH_{B}), 2.75 (s, broad, 3H, CH₃N), 2.73-2.63 (m, 1H, NCH_{B}-Pro), 2.2-1.4 (m, 8H, 4CH₂). Impurities of about 9% can be seen at 6.2, 3.7 and 1.2 ppm.
¹⁹F-NMR (D₂O, CF₃CO₂H ext. ref.) δ = -3.65 and -3.71 (2s, ratio, 45:55, 3F, CF₃), 1 AB system centered at -47.80; A: -47.44 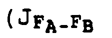 = 281 Hz); B: -48.16 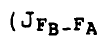 = 281 Hz), AB system centered at -48.09; A: -47.64 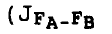 = 281 Hz), -48.53 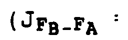 = 281 Hz) = CF₂ of the two diastereoisomers, ratio 45:55, impurities of remaining alcohol described in Step G at -6.9 and -54.0 (about 5%) and unknown structure at -3.3 and -45.5 (9%).

### EXAMPLE 2

### L-Prolinamide, N-methyl-D-phenylalanyl-N-[1-[3-[(aminoiminomethyl)amino]propyl]-3,3-difluoro-2-oxohexyl]-dihydro chloride, hydrate

### STEP A:

### N-[1-[4-[bis[(1,1-Dimethylethoxy)carbonyl]amino]methylene]amino]-1-nitrobutane

Bis-Boc-S-methylisothiourea (13.8 g, 47.6 mmol) was added at a time to a stirred solution of 4-amino-1-nitrobutane, hydrochloride [prepared according to W. Keller-Schierlein, P. Mertens, V. Prelog, and A. Walser, *Helv. Chim.* *Acta*, **48**, Fasc. 4 (1965) 710] (2.1 g, 13.6 mmol) and 6.6 ml (47.6 mmol) of NEt₃ in 40 ml of anhydrous dimethylformamide. The mixture was stirred under an atmosphere of argon for 14 hours. 200 ml of Et₂O were added and the solution washed with water and concentrated solutions of citric acid, sodium bicarbonate, and sodium chloride. The organic layer was dried over MgSO₄ and evaporated [2.666.10³ Pa (20 Torr), 30°C] to afford 15 g of a yellow oil. Flash chromatography on silica gel ( 500 g, eluent: AcOEt/petroleum ether: 1/5) and evaporation of the product-containing fractions [2.666.10³ Pa (20 Torr), 30°C and then 1.333 Pa (0.01 Torr), 20°C] afforded 3.48 g (71%) of the protected guanidino derivative as a white solid.
Rf = 0.5 (AcOEt/petroleum ether: 1/4). m.p.: 94-96°C.

### STEP B:

### N-1-[9-[bis[(1,1-Dimethylethoxy)carbonyl]amino]methylene]amino-4,4-difluoro-5-hydroxy-6-nitro-1-nonene

A mixture of the above-prepared nitroderivative (Step A, 0.48 g, 1.33 mmol) and 2,2-difluoro-pentene-1-al, ethyl hemiacetal (0.283 g, 1.7 mmol) and a catalytic amount of potassium carbonate (about 40 mg) was stirred for 14 hours at 40°C. AcOEt (50 ml) was added and the resulting mixture washed with water and brine. Drying over MgSO₄ of the organic layer and evaporation of the solvent (20 Torr, 30°C) afforded a colorless oil (0.57 g), which was applied to flash chromatography on silica gel (20 g, eluent: AcOEt/petroleum ether: 1/5). The product-containing fractions were pooled and evaporated [2.666.10³ Pa (20 Torr), 30°C and 1.333 Pa (0.01 Torr), 20°C] to give 0.332 g (52%) of the desired nitroalcohol as a slightly yellow solid.
Rf = 0.4 (AcOEt/petroleum ether: 1/3).

### STEP C:

### N-1-[9-(bis[(1,1-Dimethylethoxy)carbonyl)amino]methylene]amino-4,4-difluoro-5-hydroxy-6-aminononane

To a stirred solution of the above-prepared nitroalcohol (Step B, 0.33 g, 0.67 mmol) in i.-propanol (40 ml) was added 0.1 g of freshly prepared Raney-Nickel. The mixture was applied to hydrogenation under atmospheric pressure for 16 hours when H₂ consumption had completed. Filtration of the mixture and evaporation of the solvent [2.666.10³ Pa (20 Torr), 30°C and 1.333 Pa (0.01 Torr), 20°C] afforded the desired difluoroamino alcohol as a colorless oil. Yield: 302 mg (93%).

### STEP D:

### L-Prolinamide, N-[(1,1-dimethylethoxy)carbonyl]-N-methyl-D-phenylalanyl-N-[1-[3-[[bis[[(1,1-dimethylethoxy)carbonyl]amino]methylene]amino]propyl]-3,3-difluoro-2-hydroxyhexyl]-

Following the procedure as described for Example 1, Step G: with 0.23 g (0.61 mmol) of Boc-N-(methyl)-D-Phe-Pro-OH, 0.093 g (0.61 mmol) of HOBt, 0.126 g (0.61 mmol) of DCC, 0.08 g (0.75 mmol) of N-methylmorpholine, and 0.23 g (0.61 mmol) of the above-prepared difluoroamino alcohol (Step C), in 10 ml of CH₂Cl₂ and flash chromatography of the crude reaction product on silica gel (20 g, eluent: AcOEt/petroleum ether: 1/3-1/1) 0.24 g (49%) of the protected tripeptide analog were obtained as a colorless oil. Rf = 0.4-0.6 (AcOEt/petroleum ether : 1/1).

### STEP E:

### L-Prolinamide, N-[(1,1-dimethylethoxy)carbonyl]-N-methyl-D-phenylalanyl-N-[1-[3-[[bis[[(1,1-dimethylethoxy)carbonyl]amino]methylene]amino]propyl]-3,3-difluoro-2-oxohexane]-

The product of Step D (0.24 g, 0.29 mmol) was oxidized to the corresponding ketone following the procedure described in Example 1, Step H with the following quantities: 0.051 ml (0.59 mmol) of oxalyl chloride, 0.084 ml (1.1 mmol) of DMSO, and 0.2 ml (2 mmol) of NEt₃ in 6 ml of anhydrous CH₂Cl₂. Flash chromatography of the crude reaction product (0.19 g) on silica gel (10 g, eluent AcOEt/petroleum ether: 2/3) and evaporation of the product-containing fractions afforded 0.16 g (67%) of the desired ketone as a colorless oil.
Rf = 0.2-0.3 (two spots) (AcOEt/petroleum ether: 2/3).

### STEP F:

### L-Prolinamide, N-methyl-D-phenylalanyl-N-[1-[3-[(aminoiminomethyl)amino]propyl]-3,3-difluoro-2-oxohexyl]-dihydrochloride, hydrate

To 0.15 g (0.186 mmol) of the above-prepared ketone (Step E) was added 50 ml of a saturated HCl gas/Et₂O solution and the mixture was stirred for 48 hours. The solvent was evaporated and the solid residue dissolved in water. Filtration of the solution on a Millipore® filter disk and lyophilization of the filtrate afforded 0.11 g (100%) of the title compound as a yellow solid.
Rf = 0.4 and 0.45 (two diastereoisomers) (BuOH/AcOH/H₂O: 3/1/1).

As stated above, the compounds of this invention (IA and IB) possess the property of exhibiting significant inhibition of thrombin indicating that they are effective anti-coagulants useful for the prevention of both venous and arterial thrombotic diseases.

Using standard *in vitro* assay methodology for determining inhibitory activity of thrombin as well as anticoagulant activities in human plasma using activated thromboplastin times ( 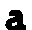 PTT) and thrombin times (TT), it is to be found that the compounds of Formulae IA and IB are effective anticoagulants.

*In vivo* anti-thrombotic effects in rats may also be utilized for evaluation. Thus, based upon standard *in vitro* and *in vivo* assay methodology it is to be found that the compounds of this invention, at doses within the range of about 2 to 50 mg per kilogram of body weight (with 2 to 20 for preferred compounds) per day will be useful in the treatment of thrombophlebitis and coronary thrombosis, as well as other venous and arterial thrombotic disease states. Of course, actual dosage and frequency will vary dependent upon the nature and severity of the condition, age, general health conditions and such other factors well known and appreciated by the skilled attending diagnostician.

In addition to the foregoing use of the novel compounds of Formulae IA and IB as inhibitors of thrombin, another aspect of this invention is the use of these compounds as effective inhibitors of human lung tryptase and, as such, are useful in the treatment of asthma.

Based upon the foregoing type assay, as well as by comparison with other compounds also known to possess tryptase inhibitory activity, it is to be found that the compounds of this invention, in their end-use application for the treatment of asthma will be about 1-100 mg per treatment; the dose regimen, of course, being dependent on the severity of the attack, the age and general condition of the patient, as well as other factors well appreciated by the attending diagnostician.

Although it may be possible that some of the administered tripeptides of this invention may survive passage through the gut following oral administration, it is preferred that the compounds be utilized via a non-oral administration. Subcutaneous, intravenous, intramuscular or intraperitoneal administrations, depot injections, implant preparations and such other non-oral methods are the preferred manners by which the compounds may be utilized. In those instances wherein asthma is being treated it is preferred to utilize metered dose aerosols or by application to the mucous membranes, (e.g. nose, throat and bronchial tubes) in an aerosol can containing a peptide derivative of this invention in the form of a spray or dry powder pharmaceutical formulation.

For a parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by Dow-Corning Corporation.

The compounds of this invention can also be administered topically. This can be accomplished by simply preparing a solution of the compound to be administered, preferably using a solvent known to promote transdermal absorption such as ethanol or dimethylsulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951, 3,797,494, 3,996,934, and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is non-porous. The pharmaceutically effective compound dissolves in and diffuses through the matrix itself. Release by microporous flow occurs when the pharmaceutically effective compound is transported through a liquid phase in the pores of the matrix.

The preferred compounds of this invention (IA and IB) which are of particular interest are those compounds specified in the following charts:

| Compounds of Formula IA | | | | | |
|---|---|---|---|---|---|
| R₁ | R₂ | P₃ | P₂ | P₁ | R₃ |
| H | CH₃ | D-Phe | L-Pro | Arg | CF₂(CH₂)₂CH₃ |
| H | H | D-Phe | L-Pro | Arg | CF₂(CH₂)₂CH₃ |
| H | CH₃ | D-Phe | L-Pro | Arg | CF₂(CH₂)₂CO₂Et |
| H | CH₃ | D-Phe | L-Pro | Arg | CF₂(CH₂)₂CONHCH₃ |
| H | CH₃ | D-Phe | L-Pro | Arg | CF₂(CH₂)₄CONHCH₃ |
| CH₃ | CH₃ | D-CHM | L-Pro | Arg | CF₂(CH₂)₂CO₂Et |
| CH₃ | CH₃ | D-CH | L-Pro | Arg | CF₂(CH₂)₂CO₂Et |
| H | CH₃ | D-CHM | L-Pro | Arg | CF₂(CH₂)₂CH₃ |
| H | CH₃ | D-CHM | L-Pro | Arg | CF₂(CH₂)₂CO₂Et |
| H | CH₃ | D-CH | L-Pro | Arg | CF₂(CH₂)₂CH₃ |

wherein CH is cyclohexyl, CHM is cyclohexylmethyl, both being the substituents on the α-carbon atom of the modified P₃-α-amino acid of the depicted tripeptide of Formula IA, and Et is ethyl.

| Compounds of Formula IB | | | |
|---|---|---|---|
| P₃ | P₂ | P₁ | R₃ |
| 2'b | L-Pro | Arg | CF₂(CH₂)₂CH₃ |
| 2'c | L-Pro | Arg | CF₂(CH₂)₂CH₃ |
| 2b | L-Pro | Arg | CF₂(CH₂)₂CH₃ |
| 2c | L-Pro | Arg | CF₂(CH₂)₂CH₃ |
| 2b | L-Pro | Arg | CF₂(CH₂)₂CO₂Et |
| 2'b | L-Pro | Arg | CF₂(CH₂)₂CO₂Et |
| 2b | L-Pro | Arg | CF₂(CH₂)₄CO₂Et |
| 2'b | L-Pro | Arg | CF₂(CH₂)₄CO₂Et |
| 2b | L-Pro | Arg | CF₂(CH₂)₃CH₃ |
| 2'b | L-Pro | Arg | CF₂(CH₂)₂CH₃ |
| 2b | L-Pro | Arg | CF₂(CH₂)₂CONHCH₃ |
| 2'b | L-Pro | Arg | CF₂(CH₂)₂CONHC₂H₅ |
| 2b | L-Pro | Arg | CF₂(CH₂)₃CO₂Et |
| 2'b | L-Pro | Arg | CF₂(CH₂)₃CONHCH₃ |

wherein the P₃ moieties 2b and 2c, and 2'b and 2'c are as previously defined, which, when combined with the carbonyl moiety to which they are attached, form the modified P₃-α-amino acid of the depicted tripeptides of Formula IB.

## Claims (Claims for the following Contracting State(s): AT, BE, CH, DE, DK, FR, GB, IT, LI, LU, MC, NL, PT, SE)

1. A compound of the formulae 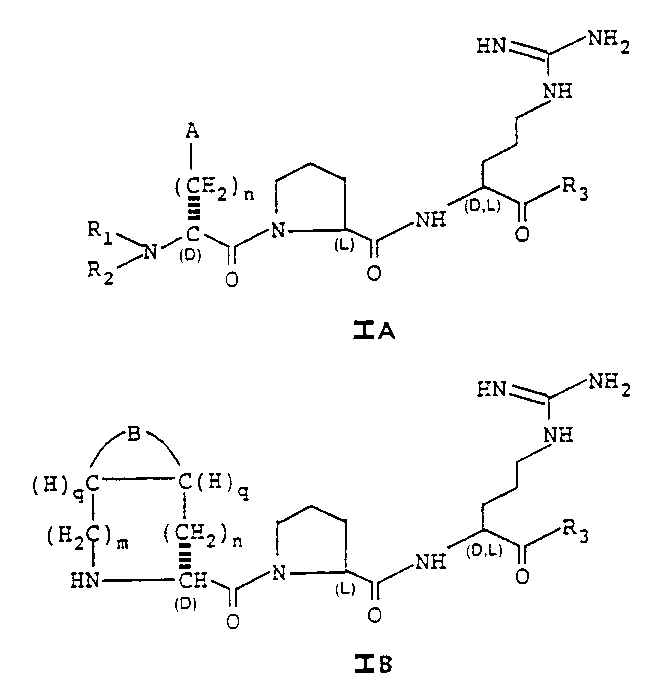 the isomers or mixtures thereof, the hydrates or the pharmaceutically acceptable salts thereof, wherein
m is zero, one or two, n is zero or one, with the proviso that the sum of m and n is less than three and greater than zero, q is zero or one with the proviso that the sum of both q's is zero or 2,
R₁ is H or C₁₋₇ alkyl,
R₂ is H or C₁₋₇ alkyl, or R₁ and R₂ taken together with the nitrogen atom to which they are attached form a 5- or 6-membered heterocycle,
R₃ is -CF₂(CH₂)ₜCH₃, -CF₂(CH₂)ₜCOOR₄, -CF₂(CH₂)ₜCONHR₄, -CF₂(CH₂)ₜCH₂OR₄ or -CF₂(CH₂)ᵥCH=CH₂, with t being 2, 3 or 4, and v is 1, 2 or 3,
R₄ is H or C₁₋₆ alkyl,
A is phenyl or cyclohexyl,
B is (CH)₄ or (CH₂)₄ which, when taken together with the two carbon atoms to which it is attached, forms a C₆-cyclic hydrocarbon moiety.

2. A compound of Claim 1 wherein the R₃ moiety is selected from the group consisting of -CF₂(CH₂)₂CH₃, -CF₂(CH₂)₂CO₂Et, -CF₂(CH₂)₂CONHCH₃ and -CF₂(CH₂)₄CONHCH₃.

3. A compound of Claim 2 having the structure of Formula IA wherein A is phenyl, n is one, R₁ is H and R₂ is C₁₋₇ alkyl.

4. A compound of Claim 3 wherein R₂ is methyl.

5. A compound of Claim 2 having the structure of Formula IA wherein A is cyclohexyl, R₁ is H and R₂ is C₁₋₇ alkyl.

6. A compound of Claim 5 wherein R₂ is methyl and n is one.

7. A compound of Claim 2 having the structure of Formula IB wherein B is(CH₂)₄, each q is zero, m is two, and n is zero.

8. A compound of Claim 2 having the structure of formula IB wherein B is (CH₂)₄, each q is zero, m is one, and n is zero or one.

9. A compound of claim 2 having the structure of formula IB wherein B is (CH₂)₄, each q is one, m is one or two and n is zero or one.

10. A compound of any one of claims 4, 7, 8 or 9 wherein R₃ is -CF₂(CH₂)₂CH₃.

11. A process for preparing a compound of the formulae 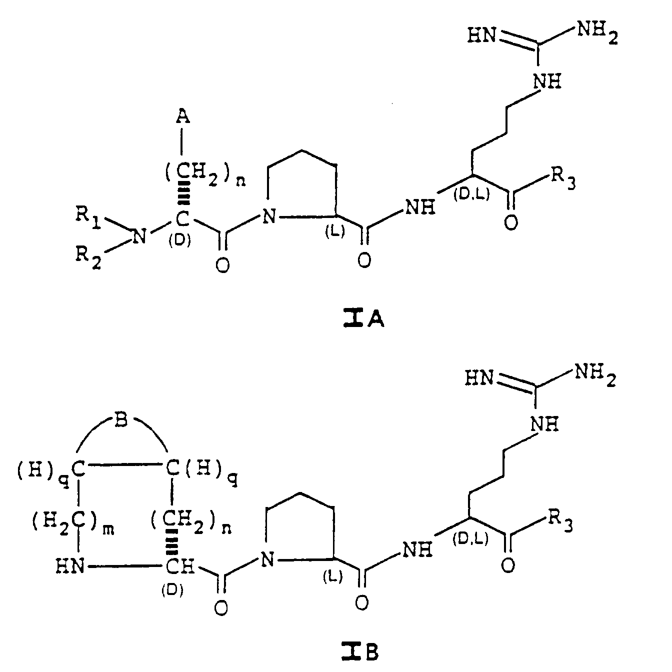 the isomers or mixtures thereof, the hydrates or the pharmaceutically acceptable salts thereof, wherein
m is zero, one or two, n is zero or one, with the proviso that the sum of m and n is less than three and greater than zero, q is zero or one with the proviso that the sum of both q's is zero or 2,
R₁ is H or C₁₋₇ alkyl,
R₂ is H or C₁₋₇ alkyl, or R₁ and R₂ taken together with the nitrogen atom to which they are attached form a 5- or 6-membered heterocycle,
R₃ is -CF₂(CH₂)ₜCH₃, -CF₂(CH₂)ₜCOOR₄, -CF₂(CH₂)ₜCONHR₄, -CF₂(CH₂)ₜCH₂OR₄ or -CF₂(CH₂)ᵥCH=CH₂, with t being 2, 3 or 4, and v is 1, 2 or 3,
R₄ is H or C₁₋₆ alkyl,
A is phenyl or cyclohexyl,
B is (CH)₄ or (CH₂)₄ which, when taken together with the two carbon atoms to which it is attached, forms a C₆-cyclic hydrocarbon moiety,
which comprises oxidizing a compound of the formulae 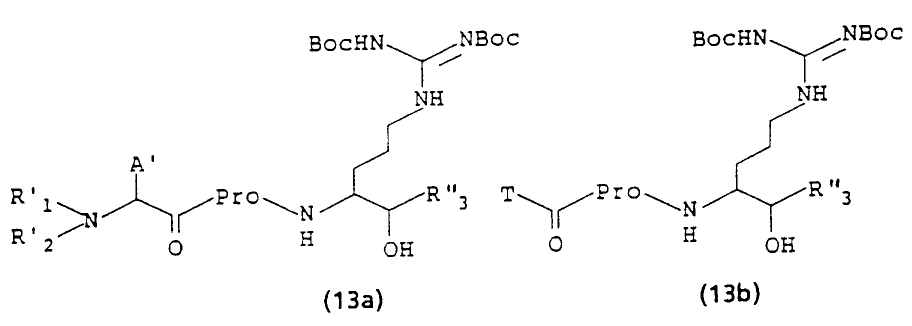 wherein
A' is 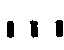 (CH₂)ₙA,
n is zero or one,
R'₁ is an N-protecting group,
R'₂ is H or C₁₋₇ alkyl,
R''₃ is -CF₂(CH₂)ₜCONHR₄, -CF₂(CH₂)ₜCH₂OR₄,
-CF₂(CH₂)ᵥCH=CH₂, or -CF₂(CH₂)ₜCOOR₄,
T is a TIC-like moiety,
t, v and R₄ being as herein above defined,
followed by removing any protecting group thereof and (a) optionally reducing any olefinic moiety of the R₃ moiety, (b) optionally N-alkylating or N-dialkylating any compound bearing a free amino group at the P₃ moiety, and (c) optionally converting the so-produced compounds to their pharmaceutically acceptable salts thereof.

12. Compounds of formula IA or IB as defined in claim 1 for use as medicine.

13. The use of the compounds of formula IA or IB as defined in claim 1, for the preparation of pharmaceutical compositions for the inhibition of thrombotic conditions.

14. The use of the compounds of formula IA or IB as defined in claim 1, for the preparation of pharmaceutical compositions for the treatment of thrombophlebitis and coronary thrombosis.

15. The use of the compounds of formula IA or IB as defined in claim 1, for the preparation of pharmaceutical compositions for the inhibiton of lung tryptase.

16. The use of the compounds of formula IA or IB as defined in claim 1, for the preparation of pharmaceutical compositions for the treatment of asthma.

17. A compound of claim 1 wherein the compound is L-prolinamide, N-methyl-D-phenyl-alanyl-N-[1-[3-[(aminoiminomethyl)amino]propyl]-3,3-difluoro-2-oxohexyl]dihydrochloride, hydrate.

## Claims (Claims for the following Contracting State(s): ES)

1. A process for preparing a compound of the formulae 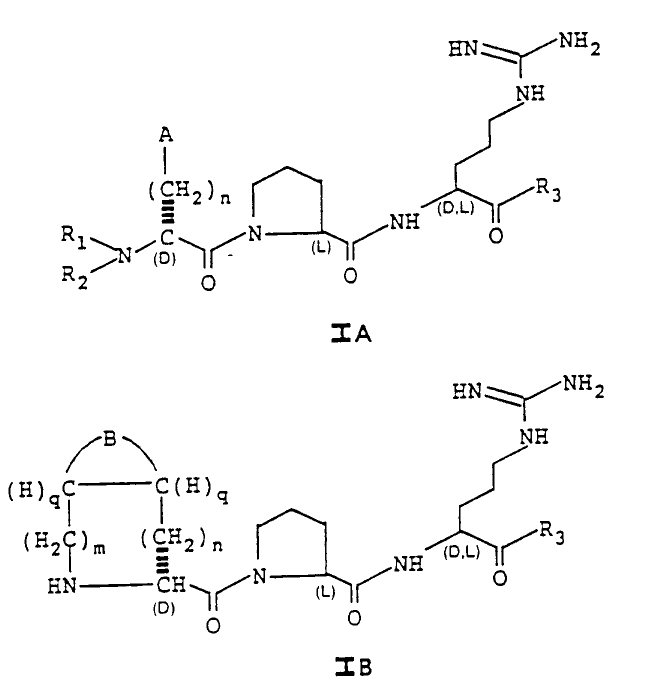 the isomers or mixtures thereof, the hydrates or the pharmaceutically acceptable salts thereof, wherein
m is zero, one or two, n is zero or one, with the proviso that the sum of m and n is less than three and greater than zero, q is zero or one with the proviso that the sum of both q's is zero or 2,
R₁ is H or C₁₋₇ alkyl,
R₂ is H or C₁₋₇ alkyl, or R₁ and R₂ taken together with the nitrogen atom to which they are attached form a 5- or 6-membered heterocycle,
R₃ is -CF₂(CH₂)ₜCH₃, -CF₂(CH₂)ₜCOOR₄, -CF₂(CH₂)ₜCONHR₄, -CF₂(CH₂)ₜCH₂OR₄ or
-CF₂(CH₂)ᵥCH=CH₂, with t being 2, 3 or 4, and v is 1, 2 or 3,
R₄ is H or C₁₋₆ alkyl,
A is phenyl or cyclohexyl,
B is (CH)₄ or (CH₂)₄ which, when taken together with the two carbon atoms to which it is attached, forms a C₆-cyclic hydrocarbon moiety,
which comprises oxidizing a compound of the formulae 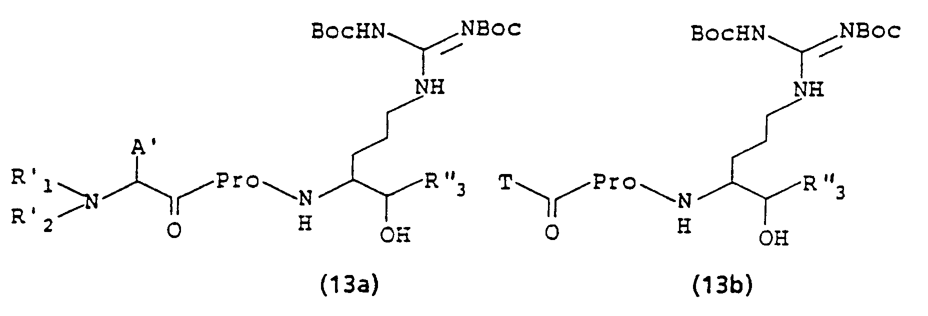 wherein
A' is 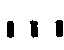 (CH₂)ₙA,
n is zero or one,
R'₁ is an N-protecting group,
R'₂ is H or C₁₋₇ alkyl,
R''₃ is -CF₂(CH₂)ₜCONHR₄, -CF₂(CH₂)ₜCH₂OR₄,
-CF₂(CH₂)ᵥCH=CH₂, or -CF₂(CH₂)ₜCOOR₄,
T is a TIC-like moiety,
t, v and R₄ being as herein above defined,
followed by removing any protecting group thereof and (a) optionally reducing any olefinic moiety of the R₃ moiety, (b) optionally N-alkylating or N-dialkylating any compound bearing a free amino group at the P₃ moiety, and (c) optionally converting the so-produced compounds to their pharmaceutically acceptable salts thereof.

2. Process according to claim 1, wherein a compound of formula (13a) or (13b) in which R''₃ is -CF₂-CH=CH-CH₂ , -CF₂(CH₂)₂CO₂Et, -CF₂(CH₂)₂CONHCH₃ or -CF₂(CH₂)₄CONHCH₃, is used for obtaining a compound of formula IA or IB in which R₃ is -CF₂(CH₂)₂CH₃ , -CF₂(CH₂)₂CO₂Et, -CF₂(CH₂)₂CONHCH₃ or -CF₂(CH₂)₄CONHCH₃.

3. Process according to claim 1, wherein a compound of formula (13a) in which A is phenyl, n is one, R'₁ is an N-protecting group, R₂ is C₁₋₇ alkyl and R''₃ is -CF₂-CH=CH-CH₂ , -CF₂(CH₂)₂CO₂Et, -CF₂(CH₂)₂CONHCH₃ or -CF₂(CH₂)₄CONHCH₃, is used for obtaining a compound of formula IA in which A is phenyl, n is one, R₁ is H and R₂ is C₁₋₇ alkyl and R₃ is -CF₂(CH₂)₂CH₃ , -CF₂(CH₂)₂CO₂Et, -CF₂(CH₂)₂CONHCH₃ or -CF₂(CH₂)₄CONHCH₃.

4. Process according to claim 3, wherein in the compound of formula (13a) R₂ is methyl.

5. Process according to claim 1, wherein a compound of formula (13a) in which A is cyclohexyl, R'₁ is a protecting group, R₂ is C₁₋₇ alkyl and R''₃ is -CF₂-CH=CH-CH₂ , -CF₂(CH₂)₂CO₂Et, -CF₂(CH₂)₂CONHCH₃ or -CF₂(CH₂)₄CONHCH₃, is used for obtaining a compound of formula IA in which A is cyclohexyl, R₁ is H and R₂ is C₁₋₇ alkyl and R₃ is -CF₂(CH₂)₂CH₃ , -CF₂(CH₂)₂CO₂Et, -CF₂(CH₂)₂CONHCH₃ or -CF₂(CH₂)₄CONHCH₃.

6. Process according to claim 5, wherein in the compound of formula (13a) R₂ is methyl and n is one.

7. Process according to claim 1, wherein a compound of formula (13b) in which B is (CH₂)₄, each q is zero, m is two, and n is zero and R''₃ is -CF₂-CH=CH-CH₂ , -CF₂(CH₂)₂CO₂Et, -CF₂(CH₂)₂CONHCH₃ or -CF₂(CH₂)₄CONHCH₃, is used for obtaining a compound of formula IB in which B is (CH₂)₄, each q is zero, m is two, and n is zero and R₃ is -CF₂(CH₂)₂CH₃ , -CF₂(CH₂)₂CO₂Et, -CF₂(CH₂)₂CONHCH₃ or -CF₂(CH₂)₄CONHCH₃.

8. Process according to claim 1 wherein a compound of formula (13b) in which B is (CH₂)₄, each q is zero, m is one, and n is zero or one and R''₃ is -CF₂-CH=CH-CH₂, -CF₂(CH₂)₂CO₂Et, -CF₂(CH₂)₂CONHCH₃ or -CF₂(CH₂)₄CONHCH₃, is used for obtaining a compound of formula IB in which B is (CH₂)₄ each q is zero, m is one, and n is zero or one and R₃ is -CF₂(CH₂)₂CH₃ , -CF₂(CH₂)₂CO₂Et, -CF₂(CH₂)₂CONHCH₃ or -CF₂(CH₂)₄CONHCH₃.

9. Process according to claim 1 wherein a compound of formula (13b) in which B is (CH₂)₄, each q is one, m is one or two, and n is zero or one and R''₃ is -CF₂-CH=CH-CH₂ , -CF₂(CH₂)₂CO₂Et, -CF₂(CH₂)₂CONHCH₃ or -CF₂(CH₂)₄CONHCH₃, is used for obtaining a compound of formula IB in which B is (CH₂)₄, each q is one, m is one or two, and n is zero or one and R₃ is -CF₂(CH₂)₂CH₃ , -CF₂(CH₂)₂CO₂Et, -CF₂(CH₂)₂CONHCH₃ or -CF₂(CH₂)₄CONHCH₃.

10. Process according to any one of claims 4, 6, 8 and 9 wherein in the compound of formula (13a) or (13b), R₃ is -CF₂(CH₂)₂CH₃.

11. Process according to claim 1 wherein a compound of formula (13a) in which A is a phenyl, n is one, R'₁ is an N-protecting group, R₂ is a methyl, and R''₃ is -CF₂CH=CH-CH₃ is used for obtaining the compound : L-prolinamide, N-methyl-D-phenyl-alanyl-N-[1-[3-[(aminoiminomethyl) amino]propyl]-3,3-difluoro-2-oxohexyl]dihydrochloride, hydrate.

12. The use of the compounds of formula IA or IB as defined in claim 1 for the preparation of medicine.

13. The use of the compounds of formula IA or IB as defined in claim 1, for the preparation of pharmaceutical compositions for the inhibition of thrombotic conditions.

14. The use of the compounds of formula IA or IB as defined in claim 1, for the preparation of pharmaceutical compositions for the treatment of thrombophlebitis and coronary thrombosis.

15. The use of the compounds of formula IA or IB as defined in claim 1, for the preparation of pharmaceutical compositions for the inhibiton of lung tryptase.

16. The use of the compounds or formula IA or IB as defined in claim 1, for the preparation of pharmaceutical compositions for the treatment of asthma.

## Claims (Claims for the following Contracting State(s): GR)

1. A compound of the formulae 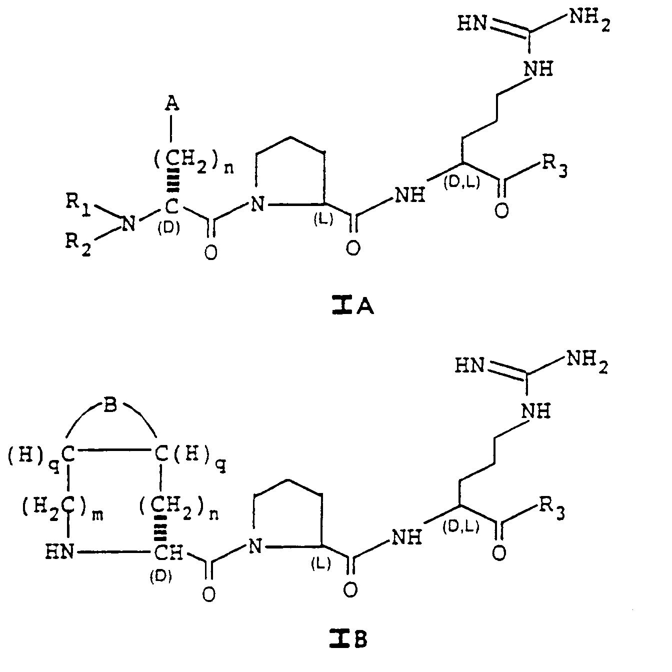 the isomers or mixtures thereof, the hydrates or the pharmaceutically acceptable salts thereof, wherein
m is zero, one or two, n is zero or one, with the proviso that the sum of m and n is less than three and greater than zero, q is zero or one with the proviso that the sum of both q's is zero or 2,
R₁ is H or C₁₋₇ alkyl,
R₂ is H or C₁₋₇ alkyl, or R₁ and R₂ taken together with the nitrogen atom to which they are attached form a 5- or 6-membered heterocycle,
R₃ is -CF₂(CH₂)ₜCH₃, -CF₂(CH₂)ₜCOOR₄, -CF₂(CH₂)ₜCONHR₄, -CF₂(CH₂)ₜCH₂OR₄ or -CF₂(CH₂)ᵥCH=CH₂, with t being 2, 3 or 4, and v is 1, 2 or 3,
R₄ is H or C₁₋₆ alkyl,
A is phenyl or cyclohexyl,
B is (CH)₄ or (CH₂)₄ which, when taken together with the two carbon atoms to which it is attached, forms a C₆-cyclic hydrocarbon moiety.

2. A compound of Claim 1 wherein the R₃ moiety is selected from the group consisting of -CF₂(CH₂)₂CH₃, -CF₂(CH₂)₂CO₂Et, -CF₂(CH₂)₂CONHCH₃ and -CF₂(CH₂)₄CONHCH₃.

3. A compound of Claim 2 having the structure of Formula IA wherein A is phenyl, n is one, R₁ is H and R₂ is C₁₋₇ alkyl.

4. A compound of Claim 3 wherein R₂ is methyl.

5. A compound of Claim 2 having the structure of Formula IA wherein A is cyclohexyl, R₁ is H and R₂ is C₁₋₇ alkyl.

6. A compound of Claim 5 wherein R₂ is methyl and n is one.

7. A compound of Claim 2 having the structure of Formula IB wherein B is(CH₂)₄, each q is zero, m is two, and n is zero.

8. A compound of Claim 2 having the structure of formula IB wherein B is(CH₂)₄, each q is zero, m is one, and n is zero or one.

9. A compound of claim 2 having the structure of formula IB wherein B is (CH₂)₄, each q is one, m is one or two and n is zero or one.

10. A compound of any one of claims 4, 7, 8 or 9 wherein R₃ is -CF₂(CH₂)₂CH₃.

11. A process for preparing a compound of the formulae 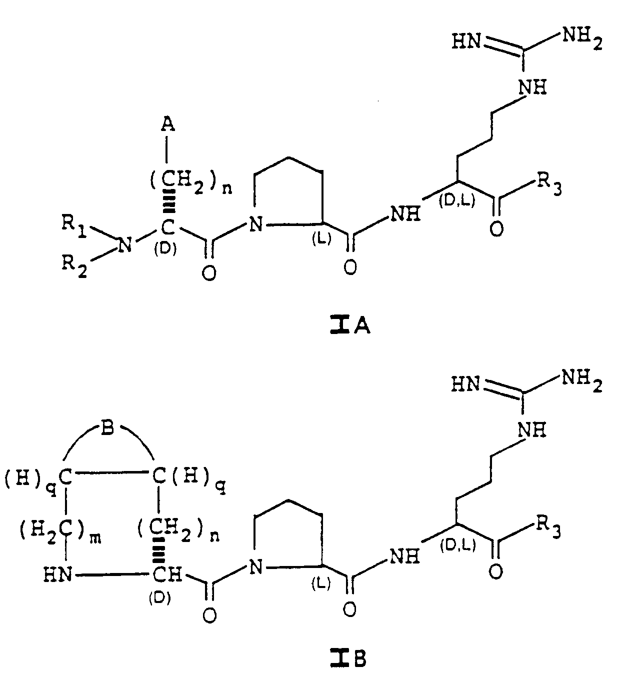 the isomers or mixtures thereof, the hydrates or the pharmaceutically acceptable salts thereof, wherein
m is zero, one or two, n is zero or one, with the proviso that the sum of m and n is less than three and greater than zero, q is zero or one with the proviso that the sum of both q's is zero or 2,
R₁ is H or C₁₋₇ alkyl,
R₂ is H or C₁₋₇ alkyl, or R₁ and R₂ taken together with the nitrogen atom to which they are attached form a 5- or 6-membered heterocycle,
R₃ is -CF₂(CH₂)ₜCH₃, -CF₂(CH₂)ₜCOOR₄, -CF₂(CH₂)ₜCONHR₄, -CF₂(CH₂)ₜCH₂OR₄ or -CF₂(CH₂)ᵥCH=CH₂, with t being 2, 3 or 4, and v is 1, 2 or 3,
R₄ is H or C₁₋₆ alkyl,
A is phenyl or cyclohexyl,
B is (CH)₄ or (CH₂)₄ which, when taken together with the two carbon atoms to which it is attached, forms a C₆-cyclic hydrocarbon moiety,
which comprises oxidizing a compound of the formulae 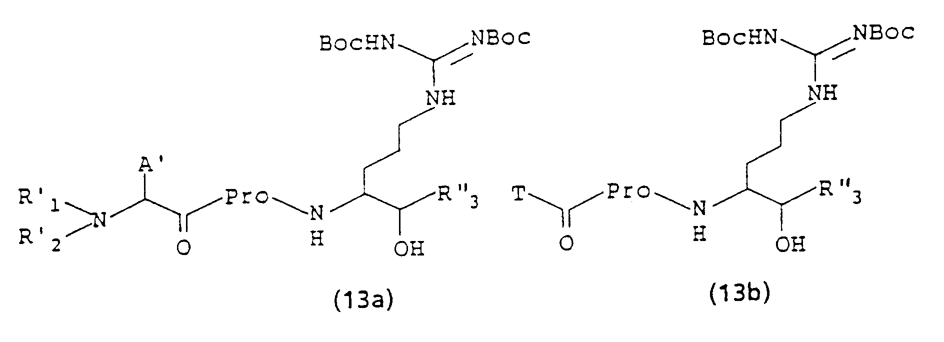 wherein
A' is 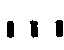 (CH₂)ₙA,
n is zero or one,
R'₁ is an N-protecting group,
R'₂ is H or C₁₋₇ alkyl,
R''₃ is -CF₂(CH₂)ₜCONHR₄, -CF₂(CH₂)ₜCH₂OR₄,
-CF₂(CH₂)ᵥCH=CH₂, or -CF₂(CH₂)ₜCOOR₄,
T is a TIC-like moiety,
t, v and R₄ being as herein above defined,
followed by removing any protecting group thereof and (a) optionally reducing any olefinic moiety of the R₃ moiety, (b) optionally N-alkylating or N-dialkylating any compound bearing a free amino group at the P₃ moiety, and (c) optionally converting the so-produced compounds to their pharmaceutically acceptable salts thereof.

12. The use of the compounds of formula IA or IB as defined in claim 1 for the preparation of medicine.

13. The use of the compounds of formula IA or IB as defined in claim 1, for the preparation of pharmaceutical compositions for the inhibition of thrombotic conditions.

14. The use of the compounds of formula IA or IB as defined in claim 1, for the preparation of pharmaceutical compositions for the treatment of thrombophlebitis and coronary thrombosis.

15. The use of the compounds of formula IA or IB as defined in claim 1, for the preparation of pharmaceutical compositions for the inhibiton of lung tryptase.

16. The use of the compounds of formula IA or IB as defined in claim 1, for the preparation of pharmaceutical compositions for the treatment of asthma.

17. A compound of claim 1 wherein the compound is L-prolinamide, N-methyl-D-phenyl-alanyl-N-[1-[3-[(aminoiminomethyl)amino]propyl]-3,3-difluoro-2-oxohexyl]dihydrochloride, hydrate.

## Patentansprüche (Patentansprüche für folgende(n) Vertragsstaat(en): AT, BE, CH, DE, DK, FR, GB, IT, LI, LU, MC, NL, PT, SE)

1. Verbindung der Formeln 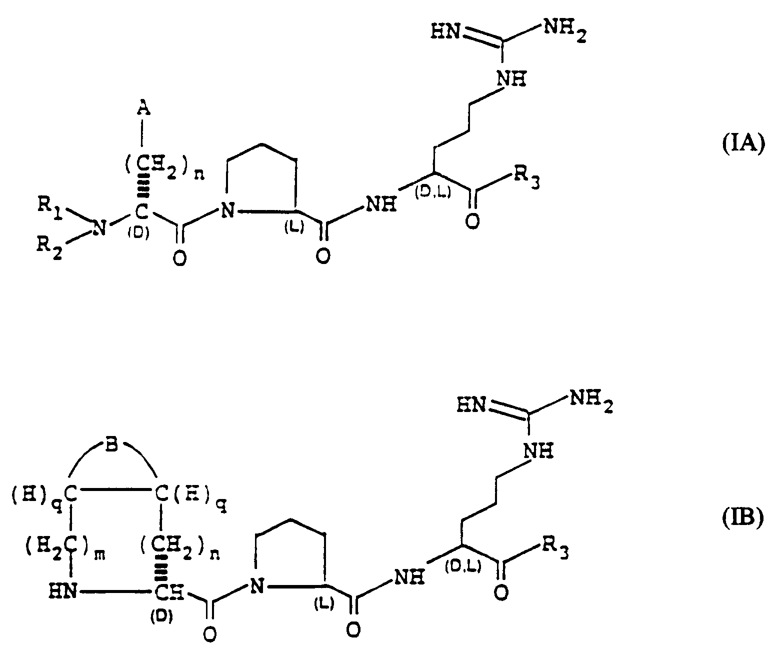 die Isomeren oder Gemische davon, die Hydrate oder die pharmazeutisch verträglichen Salze davon, wobei
m gleich 0, 1 oder 2 ist, n gleich 0 oder 1 ist, mit der Maßgabe, daß die Summe aus m und n weniger als 3 und größer als 0 ist; q gleich 0 oder 1 ist, mit der Maßgabe, daß die Summe aus beiden q-Werten gleich 0 oder 2 ist,
R₁ für ein Wasserstoffatom oder einen C₁₋₇-Alkylrest steht,
R₂ für ein Wasserstoffatom oder einen C₁₋₇-Alkylrest steht oder R₁ und R₂ zusammen mit dem Stickstoffatom, an das sie gebunden sind, einen 5- oder 6-gliedrigen Heterocyclus bilden,
R₃ für -CF₂(CH₂)ₜCH₃, -CF₂(CH₂)ₜCOOR₄, -CF₂(CH₂)ₜCONHR₄, -CF₂(CH₂)ₜCH₂OR₄ oder -CF₂(CH₂)ᵥCH=CH₂ steht, wobei t gleich 2, 3 oder 4 ist und v gleich 1, 2 oder 3 ist,
R₄ für ein Wasserstoffatom oder einen C₁₋₆-Alkylrest steht,
A eine Phenyl- oder Cyclohexylgruppe ist,
B eine (CH)₄- oder (CH₂)₄-Gruppe ist, welche zusammen mit den zwei Kohlenstoffatomen, an die sie gebunden ist, eine cyclische C₆-Kohlenwasserstoffeinheit bildet.

2. Verbindung nach Anspruch 1, wobei die R₃-Einheit ausgewählt wird aus -CF₂(CH₂)₂CH₃, -CF₂(CH₂)₂CO₂Et, -CF₂(CH₂)₂CONHCH₃ und -CF₂(CH₂)₄CONHCH₃.

3. Verbindung nach Anspruch 2 mit der Struktur der Formel IA, wobei A für eine Phenylgruppe steht, n gleich 1 ist, R₁ ein Wasserstoffatom ist und R₂ ein C₁₋₇-Alkylrest ist.

4. Verbindung nach Anspruch 3, wobei R₂ eine Methylgruppe ist.

5. Verbindung nach Anspruch 2 mit der Struktur der Formel IA, wobei A eine Cyclohexylgruppe ist, R₁ ein Wasserstoffatom ist und R₂ ein C₁₋₇-Alkylrest ist.

6. Verbindung nach Anspruch 5, wobei R₂ eine Methylgruppe ist und n gleich 1 ist.

7. Verbindung nach Anspruch 2 mit der Struktur der Formel IB, wobei B eine (CH₂)₄-Gruppe ist, jeder Wert q gleich 0 ist, m gleich 2 ist und n gleich 0 ist.

8. Verbindung nach Anspruch 2 mit der Struktur der Formel IB, wobei B eine (CH₂)₄-Gruppe ist, jeder Wert q gleich 0 ist, m gleich 1 ist und n gleich 0 oder 1 ist.

9. Verbindung nach Anspruch 2 mit der Struktur der Formel IB, wobei B eine (CH₂)₄-Gruppe ist, jeder Wert q gleich 1 ist, m gleich 1 oder 2 ist und n gleich 0 oder 1 ist.

10. Verbindung nach einem der Ansprüche 4, 7, 8 oder 9, wobei R₃ für -CF₂(CH₂)₂CH₃ steht.

11. Verfahren zur Herstellung einer Verbindung der Formeln 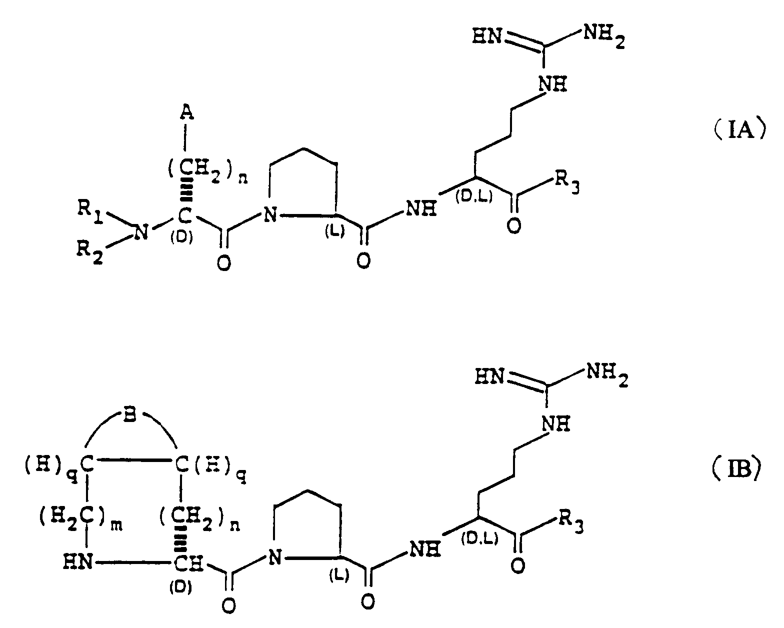 der Isomeren oder Gemische davon, der Hydrate oder der pharmazeutisch verträglichen Salze davon, wobei
m gleich 0, 1 oder 2 ist, n gleich 0 oder 1 ist, mit der Maßgabe, daß die Summe aus m und n weniger als 3 und größer als 0 ist; q gleich 0 oder 1 ist, mit der Maßgabe, daß die Summe aus beiden q-Werten gleich 0 oder 2 ist,
R₁ für ein Wasserstoffatom oder einen C₁₋₇-Alkylrest steht,
R₂ für ein Wasserstoffatom oder einen C₁₋₇-Alkylrest steht oder R₁ und R₂ zusammen mit dem Stickstoffatom, an das sie gebunden sind, einen 5- oder 6-gliedrigen Heterocyclus bilden,
R₃ für -CF₂(CH₂)ₜCH₃, -CF₂(CH₂)ₜCOOR₄, -CF₂(CH₂)ₜCONHR₄, -CF₂(CH₂)ₜCH₂OR₄ oder -CF₂(CH₂)ᵥCH=CH₂ steht, wobei t gleich 2, 3 oder 4 ist und v gleich 1, 2 oder 3 ist,
R₄ für ein Wasserstoffatom oder einen C₁₋₆-Alkylrest steht,
A eine Phenyl- oder Cyclohexylgruppe ist,
B eine (CH)₄- oder (CH₂)₄-Gruppe ist, welche zusammen mit den zwei Kohlenstoffatomen, an die sie gebunden ist, eine cyclische C₆-Kohlenwasserstoffeinheit bildet,
umfassend das Oxidieren einer Verbindung der Formeln 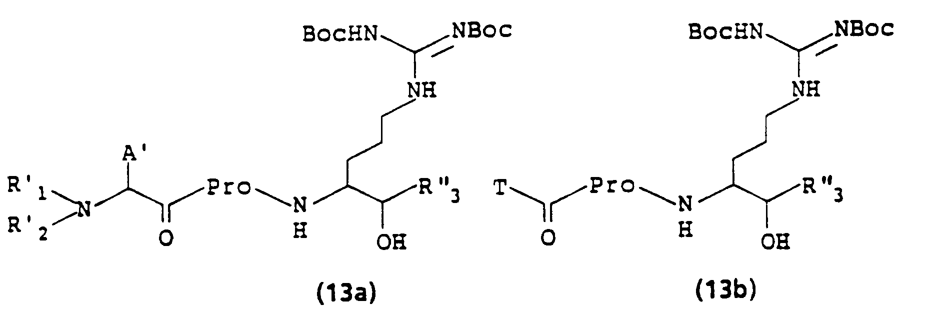 wobei
A' für 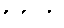 (CH₂)ₙA steht.
n gleich 0 oder 1 ist,
R'₁ eine N-Schutzgruppe ist
R'₂ ein Wasserstoffatom oder ein C₁₋₇-Alkylrest ist,
R''₃ für -CF₂(CH₂)ₜCONHR₄, -CF₂(CH₂)ₜCH₂OR₄, -CF₂(CH₂)ᵥCH=CH₂ oder
-CF₂(CH₂)ₜCOOR₄ steht,
T eine TIC-artige Einheit ist,
t, v, und R₄ wie vorstehend definiert sind,
gefolgt von dem Entfernen der Schutzgruppen davon und (a) gegebenenfalls Reduzieren der olefinischen Einheiten in der R₃-Einheit, (b) gegebenenfalls N-Alkylierung oder N-Dialkylierung der Verbindungen, die eine freie Aminogruppe an der P₃-Einheit tragen und (c) gegebenenfalls Umwandlung der so erzeugten Verbindungen zu ihren pharmazeutisch verträglichen Salzen.

12. Verbindungen der wie in Anspruch 1 definierten Formel IA oder IB zur Verwendung als Arzneistoff.

13. Verwendung der Verbindungen der wie in Anspruch 1 definierten Formel IA oder IB für die Herstellung von Arzneimitteln zur Hemmung thrombotischer Zustände.

14. Verwendung der Verbindungen der wie in Anspruch 1 definierten Formel IA oder IB für die Herstellung von Arzneimitteln zur Behandlung von Thrombophlebitis und koronarer Thrombose.

15. Verwendung der Verbindungen der wie in Anspruch 1 definierten Formel IA oder IB für die Herstellung von Arzneimitteln zur Hemmung der Lungen-Tryptase.

16. Verwendung der Verbindungen der wie in Anspruch 1 definierten Formel IA oder IB für die Herstellung von Arzneimitteln zur Behandlung von Asthma.

17. Verbindung nach Anspruch 1, wobei die Verbindung L-Prolinamid-N-methyl-D-phenyl-alanyl-N-[1-(3-[(aminoiminomethyl)amino]propyl]-3,3-difluor-2-oxohexyl]-dihydrochlorid-Hydrat ist.

## Patentansprüche (Patentansprüche für folgende(n) Vertragsstaat(en): ES)

1. Verfahren zur Herstellung einer Verbindung der Formeln 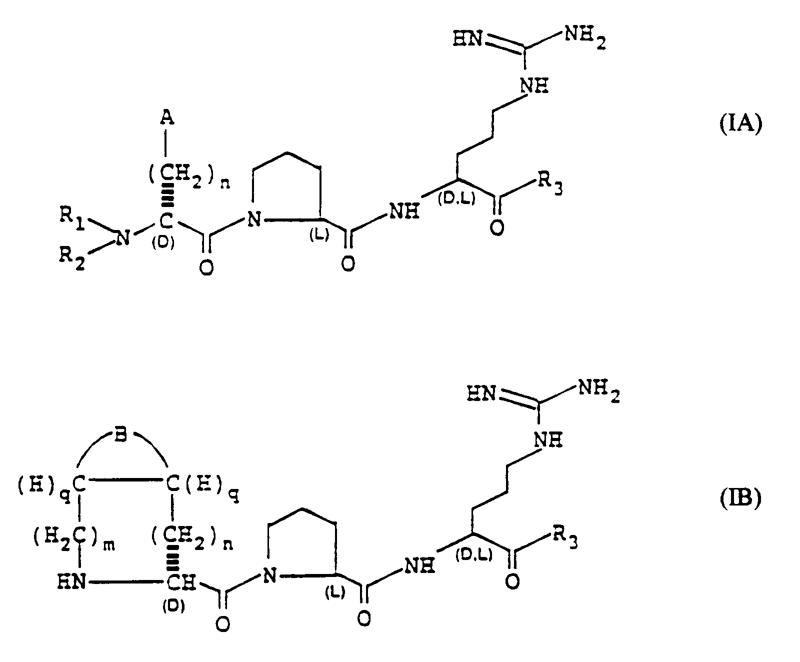 der Isomeren oder Gemische davon, der Hydrate oder der pharmazeutisch verträglichen Salze davon, wobei
m gleich 0, 1 oder 2 ist, n gleich 0 oder 1 ist, mit der Maßgabe, daß die Summe aus m und n weniger als 3 und größer als 0 ist; q gleich 0 oder 1 ist, mit der Maßgabe, daß die Summe aus beiden q-Werten gleich 0 oder 2 ist,
R₁ für ein Wasserstoffatom oder einen C₁₋₇-Alkylrest steht,
R₂ für ein Wasserstoffatom oder einen C₁₋₇-Alkylrest steht oder R₁ und R₂ zusammen mit dem Stickstoffatom, an das sie gebunden sind, einen 5- oder 6-gliedrigen Heterocyclus bilden,
R₃ für -CF₂(CH₂)ₜCH₃, -CF₂(CH₂)ₜCOOR₄, -CF₂(CH₂)ₜCONHR₄, -CF₂(CH₂)ₜCH₂OR₄ oder -CF₂(CH₂)ᵥCH=CH₂ steht, wobei t gleich 2, 3 oder 4 ist und v gleich 1, 2 oder 3 ist,
R₄ für ein Wasserstoffatom oder einen C₁₋₆-Alkylrest steht,
A eine Phenyl- oder Cyclohexylgruppe ist,
B eine (CH)₄- oder (CH₂)₄-Gruppe ist, welche zusammen mit den zwei Kohlenstoffatomen, an die sie gebunden ist, eine cyclische C₆-Kohlenwasserstoffeinheit bildet,
umfassend das Oxidieren einer Verbindung der Formeln 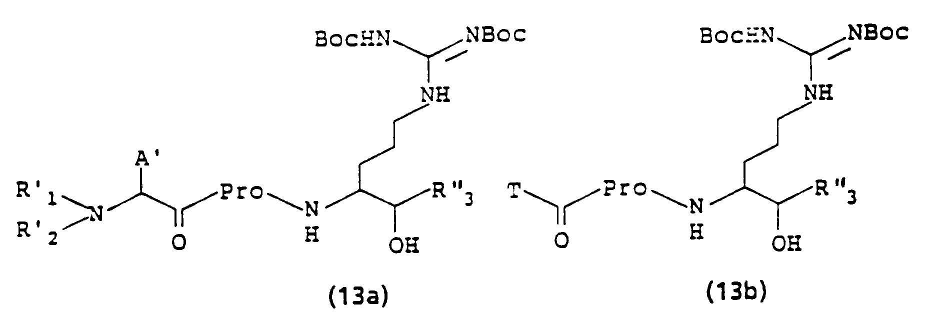 wobei
A' für 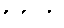 (CH₂)ₙA steht,
n gleich 0 oder 1 ist,
R'₁ eine N-Schutzgruppe ist,
R'₂ ein Wasserstoffatom oder ein C₁₋₇-Alkylrest ist,
R''₃ für -CF₂(CH₂)ₜCONHR₄, -CF₂(CH₂)ₜCH₂OR₄, -CF₂(CH₂)ᵥCH=CH₂ oder -CF₂(CH₂)ₜCOOR₄ steht,
T eine TIC-artige Einheit ist,
t, v, und R₄ wie vorstehend definiert sind,
gefolgt von dem Entfernen der Schutzgruppen davon und (a) gegebenenfalls Reduzieren der olefinischen Einheiten in der R₃-Einheit, (b) gegebenenfalls N-Alkylierung der N-Dialkylierung der Verbindungen, die eine freie Aminogruppe an der P₃-Einheit tragen und (c) gegebenenfalls Umwandlung der so erzeugten Verbindungen zu ihren pharmazeutisch verträglichen Salzen.

2. Verfahren nach Anspruch 1, wobei eine Verbindung der Formel (13a) oder (13b), in welcher R''₃ für -CF₂-CH=CH-CH₂ , -CF₂(CH₂)₂CO₂Et , -CF₂(CH₂)₂CONHCH₃ oder -CF₂(CH₂)₄CONHCH₃ steht, verwendet wird, um eine Verbindung der Formel IA oder IB zu erhalten, in der R₃ für -CF₂(CH₂)₂CH₃ , -CF₂(CH₂)₂CO₂Et , -CF₂(CH₂)₂CONHCH₃ oder -CF₂(CH₂)₄CONHCH₃ steht.

3. Verfahren nach Anspruch 1, wobei eine Verbindung der Formel (13a), in der A eine Phenylgruppe ist, n gleich 1 ist, R'₁ eine N-Schutzgruppe ist, R₂ ein C₁₋₇-Alkylrest ist und R''₃ für -CF₂-CH=CH-CH₂ , -CF₂(CH₂)₂CO₂Et , -CF₂(CH₂)₂CONHCH₃ oder -CF₂(CH₂)₄-CONHCH₃ steht, verwendet wird, um eine Verbindung der Formel IA zu erhalten, in der A eine Phenylgruppe ist, n gleich 1 ist, R₁ einWasserstoffatom ist, R₂ ein C₁₋₇-Alkylrest ist und R₃ für -CF₂(CH₂)₂CH₃ , -CF₂(CH₂)₂CO₂Et , -CF₂(CH₂)₂CONHCH₃ oder -CF₂(CH₂)₄CONHCH₃ steht.

4. Verfahren nach Anspruch 3, wobei in der Verbindung der Formel (13a) R₂ eine Methylgruppe ist.

5. Verfahren nach Anspruch 1, wobei eine Verbindung der Formel (13a), in der A eine Cyclohexylgruppe ist, R'₁ eine Schutzgruppe ist, R₂ ein C₁₋₇-Alkylrest ist und R''₃ für -CF₂-CH=CH-CH₂ , -CF₂(CH₂)₂CO₂Et , -CF₂(CH₂)₂CONHCH₃ oder -CF₂(CH₂)₄-CONHCH₃ steht, verwendet wird, um eine Verbindung der Formel IA zu erhalten, in der A eine Cyclohexylgruppe ist, R₁ einWasserstoffatom ist, R₂ ein C₁₋₇-Alkylrest ist und R₃ für -CF₂(CH₂)₂CH₃ , -CF₂(CH₂)₂CO₂Et , -CF₂(CH₂)₂CONHCH₃ oder -CF₂(CH₂)₄CONHCH₃ steht.

6. Verfahren nach Anspruch 5, wobei in der Verbindung der Formel (13a) R₂ eine Methylgruppe ist und n gleich 1 ist.

7. Verfahren nach Anspruch 1, wobei eine Verbindung der Formel (13b), in der B für eine (CH₂)₄-Gruppe steht, jeder Wert q gleich 0 ist, m gleich 2 und n gleich 0 ist und R''₃ für -CF₂-CH=CH-CH₂, -CF₂(CH₂)₂CO₂Et, -CF₂(CH₂)₂CONHCH₃ oder -CF₂(CH₂)₄CONHCH₃ steht, verwendet wird, um eine Verbindung der Formel IB zu erhalten, in der B für eine (CH₂)₄-Gruppe steht, jeder Wert q gleich 0 ist, m gleich 2 und n gleich 0 ist und R₃ für -CF₂(CH₂)₂CH₃ , -CF₂(CH₂)₂CO₂Et , -CF₂(CH₂)₂CONHCH₃ oder -CF₂(CH₂)₄CONHCH₃ steht.

8. Verfahren nach Anspruch 1, wobei eine Verbindung der Formel (13b), in der B für eine (CH₂)₄-Gruppe steht, jeder Wert q gleich 0 ist, m gleich 1 und n gleich 0 oder 1 ist und R''₃ für -CF₂-CH=CH-CH₂ , -CF₂(CH₂)₂CO₂Et , -CF₂(CH₂)₂CONHCH₃ oder -CF₂(CH₂)₄CONHCH₃ steht, verwendet wird, um eine Verbindung der Formel IB zu erhalten, in der B für eine (CH₂)₄-Gruppe steht, jeder Wert q gleich 0 ist, m gleich 1 ist, n gleich 0 oder 1 ist und R₃ für -CF₂(CH₂)₂CH₃, -CF₂(CH₂)₂CO₂Et, -CF₂(CH₂)₂CONHCH₃ oder -CF₂(CH₂)₄CONHCH₃ steht.

9. Verfahren nach Anspruch 1, wobei eine Verbindung der Formel (13b), in der B für eine (CH₂)₄-Gruppe steht, jeder Wert q gleich 1 ist, m gleich 1 oder 2 ist, n gleich 0 oder 1 ist und R''₃ für -CF₂-CH=CH-CH₂ , -CF₂(CH₂)₂CO₂Et , -CF₂(CH₂)₂CONHCH₃ oder -CF₂(CH₂)₄-CONHCH₃ steht, verwendet wird, um eine Verbindung der Formel IB zu erhalten, in der B für eine (CH₂)₄-Gruppe steht, jeder Wert q gleich 1 ist, m gleich 1 oder 2 ist, n gleich 0 oder 1 ist und R₃ für -CF₂(CH₂)₂CH₃ , -CF₂(CH₂)₂CO₂Et , -CF₂(CH₂)₂-CONHCH₃ oder -CF₂(CH₂)₄- CONHCH₃ steht.

10. Verfahren nach einem der Ansprüche 4, 6, 8 und 9, wobei in der Verbindung der Formel (13a) oder (13b) R₃ für -CF₂(CH₂)₂CH₃ steht.

11. Verfahren nach Anspruch 1, wobei eine Verbindung der Formel (13a), in der A eine Phenylgruppe ist, n gleich 1 ist, R'₁ eine N-Schutzgruppe ist, R₂ eine Methylgruppe ist und R''₃ für -CF₂-CH=CH-CH₃ steht, verwendet wird, um die Verbindung L-Prolinamid-N-methyl-D-phenyl-alanyl-N-[1-[3-[(aminoiminomethyl)amino]propyl]-3,3-difluor-2-oxohexyl]dihydrochlorid-Hydrat zu erhalten.

12. Verwendung der Verbindungen der wie in Anspruch 1 definierten Formel IA oder IB zur Herstellung von Arzneistoffen.

13. Verwendung der Verbindungen der wie in Anspruch 1 definierten Formel IA oder IB für die Herstellung von Arzneimitteln zur Hemmung thrombotischer Zustände.

14. Verwendung der Verbindungen der wie in Anspruch 1 definierten Formel IA oder IB für die Herstellung von Arzneimitteln zur Behandlung von Thrombophlebitis und koronarer Thrombose.

15. Verwendung der Verbindungen der wie in Anspruch 1 definierten Formel IA oder IB für die Herstellung von Arzneimitteln zur Hemmung der Lungen-Tryptase.

16. Verwendung der Verbindungen der wie in Anspruch 1 definierten Formel IA oder IB für die Herstellung von Arzneimitteln zur Behandlung von Asthma.

## Patentansprüche (Patentansprüche für folgende(n) Vertragsstaat(en): GR)

1. Verbindung der Formeln 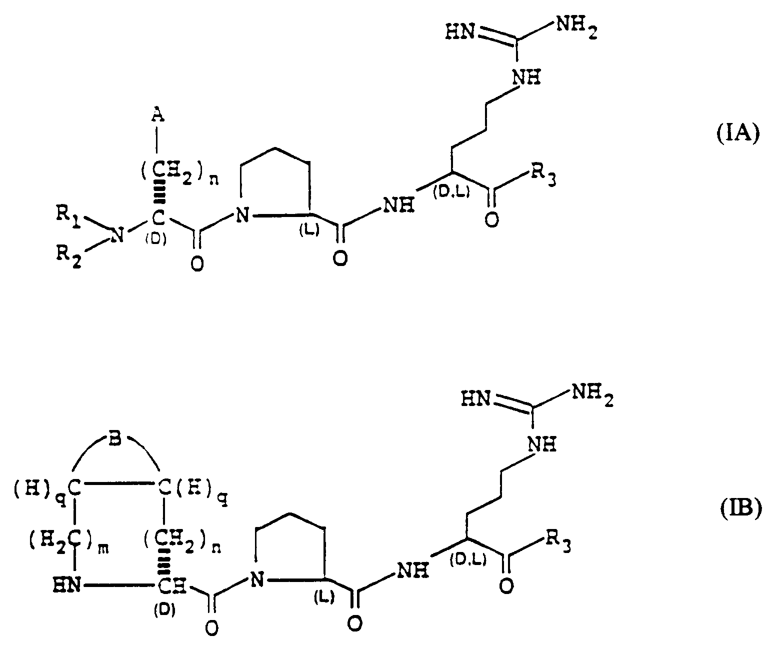 die Isomeren oder Gemische davon, die Hydrate oder die pharmazeutisch verträglichen Salze davon, wobei
m gleich 0, 1 oder 2 ist, n gleich 0 oder 1 ist, mit der Maßgabe, daß die Summe aus m und n weniger als 3 und größer als 0 ist; q gleich 0 oder 1 ist, mit der Maßgabe, daß die Summe aus beiden q-Werten gleich 0 oder 2 ist,
R₁ für ein Wasserstoffatom oder einen C₁₋₇-Alkylrest steht,
R₂ für ein Wasserstoffatom oder einen C₁₋₇-Alkylrest steht oder R₁ und R₂ zusammen mit dem Stickstoffatom, an das sie gebunden sind, einen 5- oder 6-gliedrigen Heterocyclus bilden,
R₃ für -CF₂(CH₂)ₜCH₃, -CF₂(CH₂)ₜCOOR₄, -CF₂(CH₂)ₜCONHR₄, -CF₂(CH₂)ₜCH₂OR₄ oder -CF₂(CH₂)ᵥCH=CH₂ steht, wobei t gleich 2, 3 oder 4 ist und v gleich 1, 2 oder 3 ist,
R₄ für ein Wasserstoffatom oder einen C₁₋₆-Alkylrest steht,
A eine Phenyl- oder Cyclohexylgruppe ist,
B eine (CH)₄- oder (CH₂)₄-Gruppe ist, welche zusammen mit den zwei Kohlenstoffatomen, an die sie gebunden ist, eine cyclische C₆-Kohlenwasserstoffeinheit bildet.

2. Verbindung nach Anspruch 1, wobei die R₃-Einheit ausgewählt wird aus -CF₂(CH₂)₂CH₃, -CF₂(CH₂)₂CO₂Et, -CF₂(CH₂)₂CONHCH₃ und -CF₂(CH₂)₄CONHCH₃.

3. Verbindung nach Anspruch 2 mit der Struktur der Formel IA, wobei A für eine Phenylgruppe steht, n gleich 1 ist, R₁ ein Wasserstoffatom ist und R₂ ein C₁₋₇-Alkylrest ist.

4. Verbindung nach Anspruch 3, wobei R₂ eine Methylgruppe ist.

5. Verbindung nach Anspruch 2 mit der Struktur der Formel IA, wobei A eine Cyclohexylgruppe ist, R₁ ein Wasserstoffatom ist und R₂ ein C₁₋₇- Alkylrest ist.

6. Verbindung nach Anspruch 5, wobei R₂ eine Methylgruppe ist und n gleich 1 ist.

7. Verbindung nach Anspruch 2 mit der Struktur der Formel IB, wobei B eine (CH₂)₄-Gruppe ist, jeder Wert q gleich 0 ist, m gleich 2 ist und n gleich 0 ist.

8. Verbindung nach Anspruch 2 mit der Struktur der Formel IB, wobei B eine (CH₂)₄-Gruppe ist, jeder Wert q gleich 0 ist, m gleich 1 ist und n gleich 0 oder 1 ist.

9. Verbindung nach Anspruch 2 mit der Struktur der Formel IB, wobei B eine (CH₂)₄-Gruppe ist, jeder Wert q gleich 1 ist, m gleich 1 oder 2 ist und n gleich 0 oder 1 ist.

10. Verbindung nach einem der Ansprüche 4, 7, 8 oder 9, wobei R₃ für -CF₂(CH₂)₂CH₃ steht.

11. Verfahren zur Herstellung einer Verbindung der Formeln 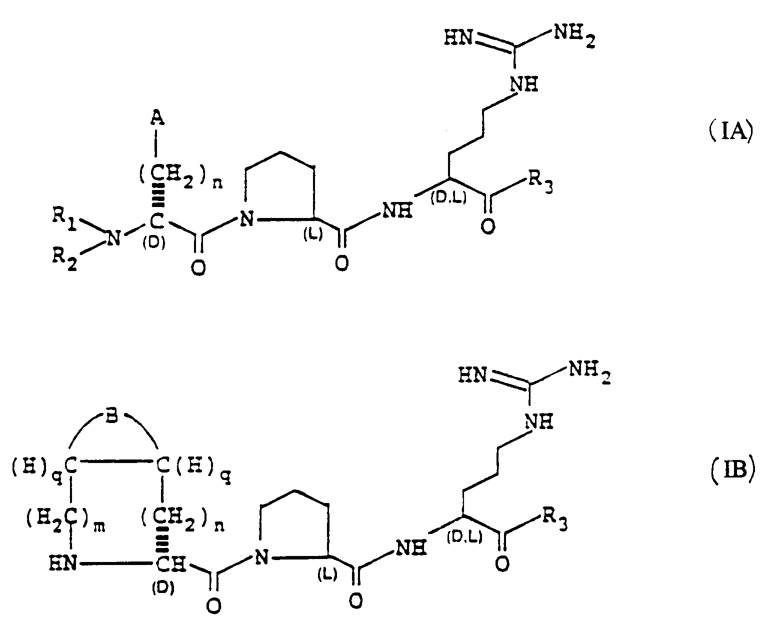 der Isomeren oder Gemische davon, der Hydrate oder der pharmazeutisch verträglichen Salze davon, wobei
m gleich 0, 1 oder 2 ist, n gleich 0 oder 1 ist, mit der Maßgabe, daß die Summe aus m und n weniger als 3 und größer als 0 ist; q gleich 0 oder 1 ist, mit der Maßgabe, daß die Summe aus beiden q-Werten gleich 0 oder 2 ist,
R₁ für ein Wasserstoffatom oder einen C₁₋₇-Alkylrest steht,
R₂ für ein Wasserstoffatom oder einen C₁₋₇-Alkylrest steht oder R₁ und R₂ zusammen mit dem Stickstoffatom, an das sie gebunden sind, einen 5- oder 6-gliedrigen Heterocyclus bilden,
R₃ für -CF₂(CH₂)ₜCH₃, -CF₂(CH₂)ₜCOOR₄, -CF₂(CH₂)ₜCONHR₄, -CF₂(CH₂)ₜCH₂OR₄ oder -CF₂(CH₂)ᵥCH=CH₂ steht, wobei t gleich 2, 3 oder 4 ist und v gleich 1, 2 oder 3 ist,
R₄ für ein Wasserstoffatom oder einen C₁₋₆-Alkylrest steht,
A eine Phenyl- oder Cyclohexylgruppe ist,
B eine (CH)₄- oder (CH₂)₄-Gruppe ist, welche zusammen mit den zwei Kohlenstoffatomen, an die sie gebunden ist, eine cyclische C₆-Kohlenwasserstoffeinheit bildet,
umfassend das Oxidieren einer Verbindung der Formeln 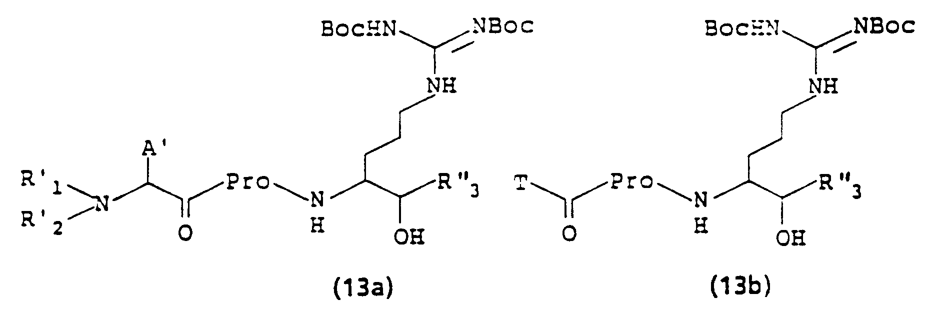 wobei
A' für 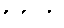 (CH₂)ₙA steht,
n gleich 0 oder 1 ist,
R'₁ eine N-Schutzgruppe ist
R'₂ ein Wasserstoffatom oder ein C₁₋₇-Alkylrest ist.
R''₃ für -CF₂(CH₂)ₜCONHR₄, -CF₂(CH₂)ₜCH₂OR₄, -CF₂(CH₂)ᵥCH=CH₂ oder
-CF₂(CH₂)ₜCOOR₄ steht,
T eine TIC-artige Einheit ist,
t, v, und R₄ wie vorstehend definiert sind,
gefolgt von dem Entfernen der Schutzgruppen davon und (a) gegebenenfalls Reduzieren der olefinischen Einheiten in der R₃-Einheit, (b) gegebenenfalls N-Alkylierung oder N-Dialkylierung der Verbindungen, die eine freie Aminogruppe an der P₃-Einheit tragen und (c) gegebenenfalls Umwandlung der so erzeugten Verbindungen zu ihren pharmazeutisch verträglichen Salzen.

12. Verwendung der Verbindungen der wie in Anspruch 1 definierten Formel IA oder IB zur Herstellung von Arzneistoffen.

13. Verwendung der Verbindungen der wie in Anspruch 1 definierten Formel IA oder IB für die Herstellung von Arzneimitteln zur Hemmung thrombotischer Zustande.

14. Verwendung der Verbindungen der wie in Anspruch 1 definierten Formel IA oder IB für die Herstellung von Arzneimitteln zur Behandlung von Thrombophlebitis und koronarer Thrombose.

15. Verwendung der Verbindungen der wie in Anspruch 1 definierten Formel IA oder IB für die Herstellung von Arzneimitteln zur Hemmung der Lungen-Tryptase.

16. Verwendung der Verbindungen der wie in Anspruch 1 definierten Formel IA oder IB für die Herstellung von Arzneimitteln zur Behandlung von Asthma.

17. Verbindung nach Anspruch 1, wobei die Verbindung L-Prolinamid-N-methyl-D-phenyl-alanyl-N-[1-(3-[(aminoiminomethyl)amino]propyl]-3,3-difluor-2-oxohexyl]-dihydrochlorid-Hydrat ist.

## Revendications (Revendications pour l'(les) Etat(s) contractant(s) suivant(s): AT, BE, CH, DE, DK, FR, GB, IT, LI, LU, MC, NL, PT, SE)

1. Composé répondant aux formules 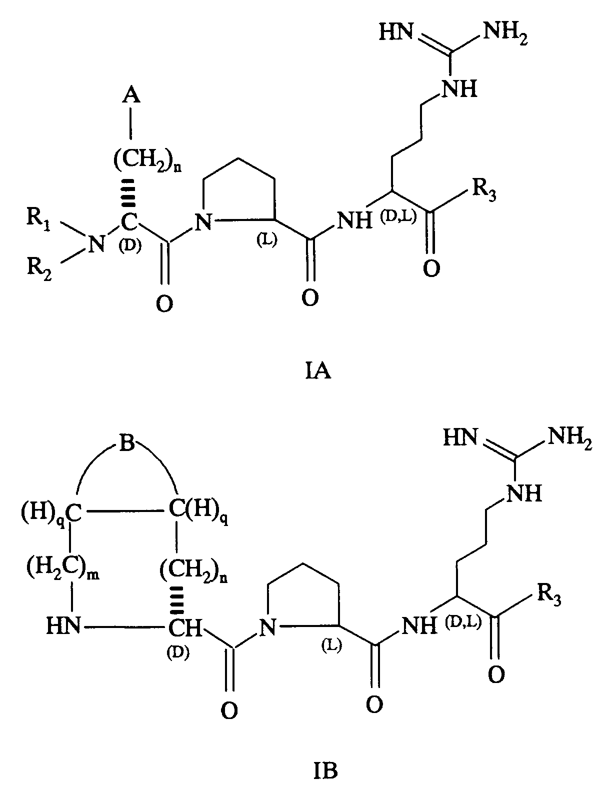 ses isomères ou mélanges, ses hydrates ou ses sels pharmaceutiquement acceptables, où
m est zéro, un ou deux, n est zéro ou un, à condition que la somme de m et n soit inférieure à trois et supérieure à zéro, q est zéro ou un, à condition que la somme des deux q soit zéro ou 2,
R₁ est H ou alkyle en C₁₋₇,
R₂ est H ou alkyle en C₁₋₇, ou bien R₁ et R₂ forment avec l'atome d'azote auquel ils sont liés un hétérocycle à 5 ou 6 chaînons,
R₃ est -CF₂(CH₂)ₜCH₃, -CF₂(CH₂)ₜCOOR₄, -CF₂(CH₂)ₜCONHR₄,
-CF₂(CH₂)ₜCH₂OR₄ ou -CF₂(CH₂)ᵥCH=CH₂, t étant 2, 3 ou 4 et v étant 1, 2 ou 3,
R₄ est H ou alkyle en C₁₋₆,
A est phényle ou cyclohexyle,
B est (CH)₄ ou (CH₂)₄ qui, lorsqu'il est combiné aux deux atomes de carbone auxquels il est lié, forme une entité hydrocarbonée cyclique en C₆.

2. Composé selon la revendication 2 dans lequel l'entité R₃ est choisie dans le groupe consistant en -CF₂(CH₂)₂CH₃, -CF₂(CH₂)₂CO₂Et, -CF₂(CH₂)₂CONHCH₃ et -CF₂(CH₂)₄CONHCH₃.

3. Composé selon la revendication 2 ayant la structure de la formule IA où A est phényle, n est un, R₁ est H et R₂ est alkyle en C₁₋₇.

4. Composé selon la revendication 3 dans lequel R₂ est méthyle.

5. Composé selon la revendication 2 ayant la structure de la formule IA où A est cyclohexyle, R₁ est H et R₂ est alkyle en C₁₋₇.

6. Composé selon la revendication 5 dans lequel R₂ est méthyle et n est un.

7. Composé selon la revendication 2 ayant la structure de la formule IB où B est (CH₂)₄, chaque q est zéro, m est deux et n est zéro.

8. Composé selon la revendication 2 ayant la structure de la formule IB où B est (CH₂)₄, chaque q est zéro, m est un et n est zéro ou un.

9. Composé selon la revendication 2 ayant la structure de la formule IB où B est (CH₂)₄, chaque q est un, m est un ou deux et n est zéro ou un.

10. Composé selon l'une quelconque des revendications 4, 7, 8 ou 9 dans lequel R₃ est -CF₂(CH₂)₂CH₃.

11. Procédé de préparation d'un composé répondant aux formules 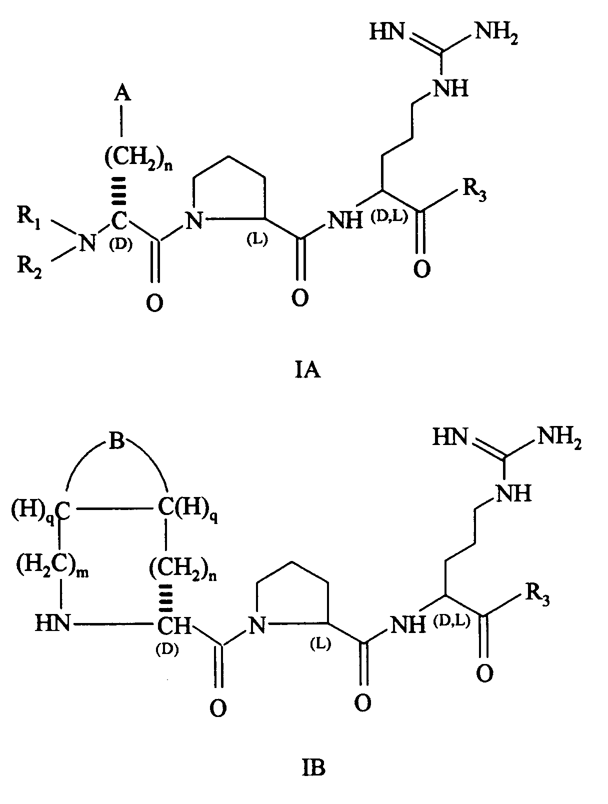 de ses isomères ou mélanges, de ses hydrates ou de ses sels pharmaceutiquement acceptables, où
m est zéro, un ou deux, n est zéro ou un, à condition que la somme de m et n soit inférieure à trois et supérieure à zéro, q est zéro ou un, à condition que la somme des deux q soit zéro ou 2,
R₁ est H ou alkyle en C₁₋₇,
R₂ est H ou alkyle en C₁₋₇, ou bien R₁ et R₂ forment avec l'atome d'azote auquel ils sont liés un hétérocycle à 5 ou 6 chaînons,
R₃ est -CF₂(CH₂)ₜCH₃, -CF₂(CH₂)ₜCOOR₄, -CF₂(CH₂)ₜCONHR₄,
-CF₂(CH₂)ₜCH₂OR₄ ou -CF₂(CH₂)ᵥCH=CH₂, t étant 2, 3 ou 4 et v étant 1, 2 ou 3,
R₄ est H ou alkyle en C₁₋₆,
A est phényle ou cyclohexyle,
B est (CH)₄ ou (CH₂)₄ qui, lorsqu'il est combiné aux deux atomes de carbone auxquels il est lié, forme une entité hydrocarbonée cyclique en C₆,
qui comprend l'oxydation d'un composé répondant aux formules 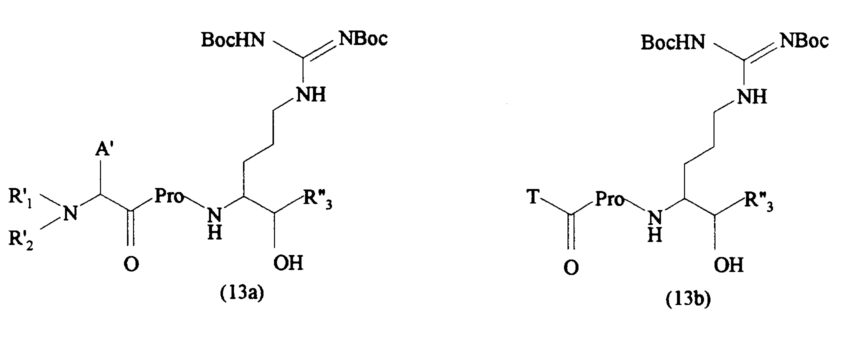 où
A' est 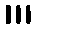 (CH₂)ₙA,
n est zéro ou un,
R'₁ est un groupe N-protecteur,
R'₂ est H ou alkyle en C₁₋₇,
R''₃ est -CF₂(CH₂)ₜCONHR₄, -CF₂(CH₂)ₜCH₂OR₄,
-CF₂(CH₂)ᵥCH=CH₂ ou -CF₂(CH₂)ₜCOOR₄,
T est une entité analogue à TIC,
t, v et R₄, étant tels que définis ci-dessus,
puis le retrait de tout groupe protecteur et (a) éventuellement la réduction de toute entité oléfinique de l'entité R₃, (b) éventuellement la N-alkylation ou la N-dialkylation de tout composé portant un groupe amino libre au niveau de l'entité P₃, et (c) éventuellement la conversion des composés ainsi produits en leurs sels pharmaceutiquement acceptables.

12. Composés de formule IA ou IB tels que définis dans la revendication 1 destinés à être utilisés comme médicament.

13. Utilisation des composés de formule IA ou IB tels que définis dans le revendication 1 pour la préparation de compositions pharmaceutiques pour l'inhibition des états thrombotiques.

14. Utilisation des composés de formule IA ou IB tels que définis dans la revendication 1 pour la préparation de compositions pharmaceutiques pour le traitement de la thrombophlébite et de la thrombose coronarienne.

15. Utilisation des composés de formule IA ou IB tels que définis dans la revendication 1 pour la préparation de compositions pharmaceutiques pour l'inhibition de la tryptase pulmonaire.

16. Utilisation des composés de formule IA ou IB tels que définis dans la revendication 1 pour la préparation de compositions pharmaceutiques pour le traitement de l'asthme.

17. Composé selon la revendication 1, le composé étant le L-prolinamide, N-méthyl-D-phényl-alanyl-N-[1-[3-[(aminoiminométhyl)amino]propyl]-3,3-difluoro-2-oxohexyl]-dichlorhydrate, hydraté.

## Revendications (Revendications pour l'(les) Etat(s) contractant(s) suivant(s): ES)

1. Procédé de préparation d'un composé répondant aux formules 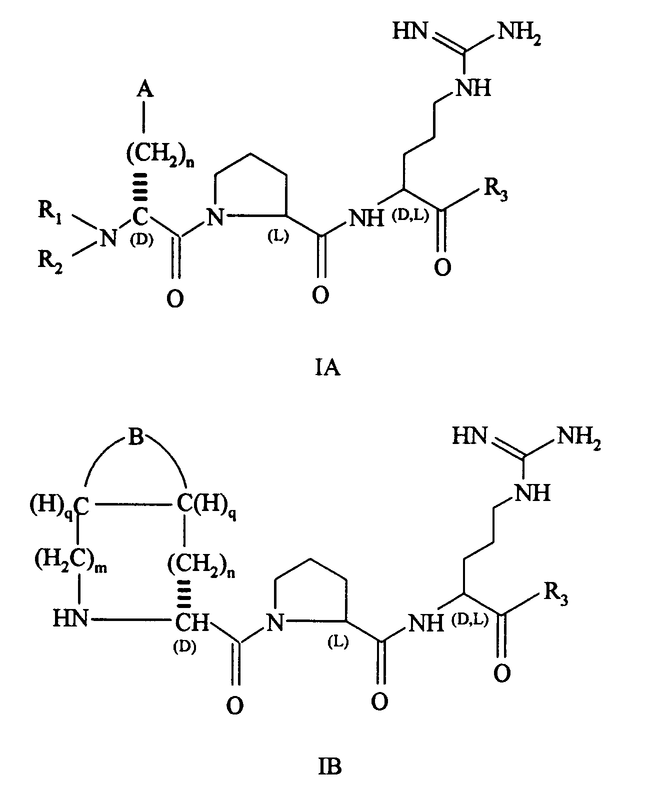 de ses isomères ou mélanges, de ses hydrates ou de ses sels pharmaceutiquement acceptables, où
m est zéro, un ou deux, n est zéro ou un, à condition que la somme de m et n soit inférieure à trois et supérieure à zéro, q est zéro ou un, à condition que la somme des deux q soit zéro ou 2,
R₁ est H ou alkyle en C₁₋₇,
R₂ est H ou alkyle en C₁₋₇, ou bien R₁ et R₂ forment avec l'atome d'azote auquel ils sont liés un hétérocycle à 5 ou 6 chaînons,
R₃ est -CF₂(CH₂)ₜCH₃, -CF₂(CH₂)ₜCOOR₄, -CF₂(CH₂)ₜCONHR₄,
-CF₂(CH₂)ₜCH₂OR₄ ou -CF₂(CH₂)ᵥCH=CH₂, t étant 2, 3 ou 4 et v étant 1, 2 ou 3,
R₄ est H ou alkyle C₁₋₆,
A est phényle ou cyclohexyle,
B est (CH)₄ ou (CH₂)₄ qui, lorsqu'il est combiné aux deux atomes de carbone auxquels il est lié, forme une entité hydrocarbonée cyclique en C₆,
qui comprend l'oxydation d'un composé répondant aux formules 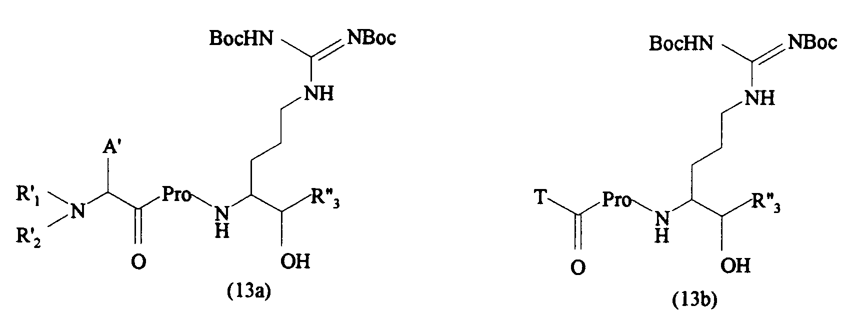 où
A' est 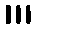 (CH₂)ₙA,
n est zéro ou un,
R'₁ est un groupe N-protecteur,
R'₂ est H ou alkyle en C₁₋₇,
R''₃ est -CF₂(CH₂)ₜCONHR₄, -CF₂(CH₂)ₜCH₂OR₄,
-CF₂(CH₂)ᵥCH=CH₂ ou -CF₂(CH₂)ₜCOOR₄,
T est une entité analogue à TIC,
t, v et R₄ étant tels que définis ci-dessus,
puis le retrait de tout groupe protecteur et (a) éventuellement la réduction de toute entité oléfinique de l'entité R₃, (b) éventuellement la N-alkylation ou la N-dialkylation de tout composé portant un groupe amino libre au niveau de l'entité P₃, et (c) éventuellement la conversion des composés ainsi produits en leurs sels pharmaceutiquement acceptables.

2. Procédé selon la revendication 1 dans lequel un composé de formule (13a) ou (13b) où R''₃ est -CF₂-CH=CH-CH₂, -CF₂(CH₂)₂CO₂Et, -CF₂(CH₂)₂CONHCH₃ ou -CF₂(CH₂)₄CONHCH₃ est utilisé pour obtenir un composé de formule IA ou IB dans lequel R₃ est -CF₂(CH₂)₂CH₃, -CF₂(CH₂)₂CO₂Et, -CF₂(CH₂)₂CONHCH₃ ou -CF₂(CH₂)₄CONHCH₃

3. Procédé selon la revendication 1 dans lequel un composé de formule (13a) où A est phényle, n est un, R'₁ est un groupe N-protecteur, R₂ est alkyle en C₁₋₇ et R''₃ est -CF₂-CH=CH-CH₂, -CF₂(CH₂)₂CO₂Et, -CF₂(CH₂)₂CONHCH₃ ou -CF₂(CH₂)₄CONHCH₃ est utilise, pour obtenir un composé de formule IA où A est phényle, n est un, R₁ est H et R₂ est alkyle en C₁₋₇ et R₃ est -CF₂(CH₂)₂CH₃, -CF₂(CH₂)₂CO₂Et, -CF₂(CH₂)₂CONHCH₃ ou -CF₂(CH₂)₄CONHCH₃.

4. Procédé selon la revendication 3 dans lequel, dans le composé de formule (13a), R₂ est méthyle.

5. Procédé selon la revendication 1 dans lequel un composé de formule (13a) où A est cyclohexyle, R'₁ est un groupe protecteur, R₂ est alkyle en C₁₋₇ et R''₃ est -CF₂-CH=CH-CH₂, -CF₂(CH₂)₂CO₂Et, -CF₂(CH₂)₂CONHCH₃ ou -CF₂(CH₂)₄CONHCH₃ est utilisé pour obtenir un composé de formule IA où A est cyclohexyle, R₁ est H et R₂ est alkyle en C₁₋₇ et R₃ est -CF₂(CH₂)₂CH₃, -CF₂(CH₂)₂CO₂Et, -CF₂(CH₂)₂CONHCH₃ ou -CF₂(CH₂)₄CONHCH₃.

6. Procédé selon la revendication 5 dans lequel, dans le composé de formule (13a), R₂ est méthyle et n est un.

7. Procédé selon la revendication 1 dans lequel un composé de formule (13b) où B est (CH₂)₄, chaque q est zéro, m est deux et n est zéro et R''₃ est -CF₂-CH=CH-CH₂, -CF₂(CH₂)₂CO₂Et, -CF₂(CH₂)₂CONHCH₃ ou -CF₂(CH₂)₄CONHCH₃ est utilisé pour obtenir un composé de formule IB où B est (CH₂)₄, chaque q est zéro, m est deux et n est zéro et R₃ est -CF₂(CH₂)₂CH₃, -CF₂(CH₂)₂CO₂Et, -CF₂(CH₂)₂CONHCH₃ ou -CF₂(CH₂)₄CONHCH₃.

8. Procédé selon la revendication 1 dans lequel un composé de formule (13b) où B est (CH₂)₄, chaque q est zéro, m est un et n est zéro ou un et R''₃ est -CF₂-CH=CH-CH₂, -CF₂(CH₂)₂CO₂Et, -CF₂(CH₂)₂CONHCH₃ ou -CF₂(CH₂)₄CONHCH₃ est utilisé pour obtenir un composé de formule IB où B est (CH₂)₄, chaque q est zéro, m est un, et n est zéro ou un et R₃ est -CF₂(CH₂)₂CH₃, -CF₂(CH₂)₂CO₂Et, -CF₂(CH₂)₂CONHCH₃ ou -CF₂(CH₂)₄CONHCH₃.

9. Procédé selon la revendication 1 dans lequel un composé de formule (13b) où B est (CH₂)₄, chaque q est un, m est un ou deux, et n est zéro ou un et R''₃ est -CF₂-CH=CH-CH₂, -CF₂(CH₂)₂CO₂Et, -CF₂(CH₂)₂CONHCH₃ ou -CF₂(CH₂)₄CONHCH₃ est utilisé pour obtenir un composé de formule IB où B est (CH₂)₄, chaque q est un, m est un ou deux, et n est zéro ou un et R₃ est -CF₂(CH₂)₂CH₃, -CF₂(CH₂)₂CO₂Et, -CF₂(CH₂)₂CONHCH₃ ou -CF₂(CH₂)₄CONHCH₃.

10. Procédé selon l'une quelconque des revendications 4, 6, 8 et 9 dans lequel, dans le composé de formule (13a) ou (13b), R₃ est -CF₂(CH₂)₂CH₃.

11. Procédé selon la revendication 1 dans lequel un composé de formule (13a) où A est un phényle, n est un, R'₁ est un groupe N-protecteur, R₂ est un méthyle et R''₃ est -CF₂CH=CH-CH₃ est utilisé pour obtenir le composé : L-prolinamide, N-méthyl-D-phényl-alanyl-N-[1-[3-[(aminoiminométhyl)amino]propyl]-3,3-difluoro-2-oxohexyl]-dichlorhydrate, hydraté.

12. Utilisation des composés de formule IA ou IB tels que définis dans la revendication 1 pour la préparation d'un médicament.

13. Utilisation des composés de formule IA ou IB tels que définis dans le revendication 1 pour la préparation de compositions pharmaceutiques pour l'inhibition des états thrombotiques.

14. Utilisation des composés de formule IA ou IB tels que définis dans la revendication 1 pour la préparation de compositions pharmaceutiques pour le traitement de la thrombophlébite et de la thrombose coronarienne.

15. Utilisation des composés de formule IA ou IB tels que définis dans la revendication 1 pour la préparation de compositions pharmaceutiques pour l'inhibition de la tryptase pulmonaire.

16. Utilisation des composés des formules IA ou IB tels que définis dans la revendication 1 pour la préparation de compositions pharmaceutiques pour le traitement de l'asthme.

## Revendications (Revendications pour l'(les) Etat(s) contractant(s) suivant(s): GR)

1. Composé répondant aux formules 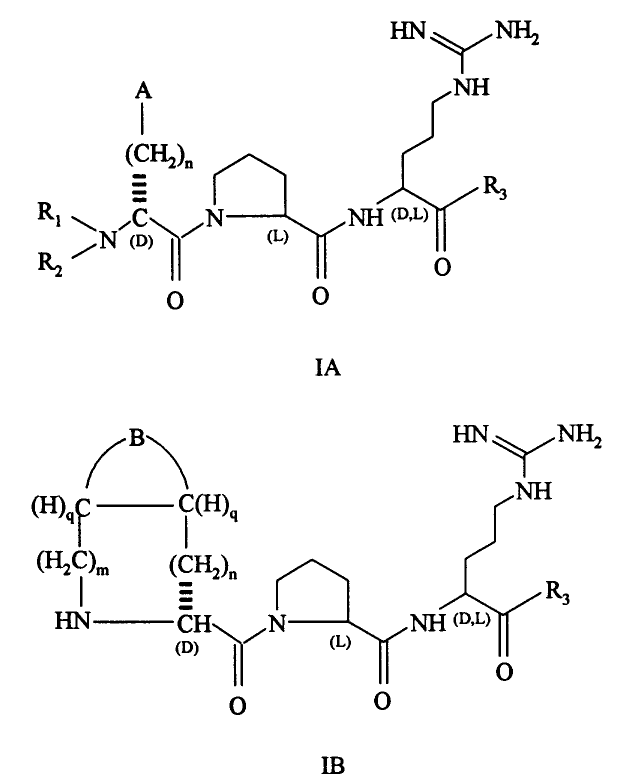 ses isomères ou mélanges, ses hydrates ou ses sels pharmaceutiquement acceptables, où
m est zéro, un ou deux, n est zéro ou un, à condition que la somme de m et n soit inférieure à trois et supérieure à zéro, q est zéro ou un, à condition que la somme des deux q soit zéro ou 2,
R₁ est H ou alkyle en C₁₋₇,
R₂ est H ou alkyle en C₁₋₇, ou bien R₁ et R₂ forment avec l'atome d'azote auquel ils sont liés un hétérocycle à 5 ou 6 chaînons,
R₃ est -CF₂(CH₂)ₜCH₃, -CF₂(CH₂)ₜCOOR₄, -CF₂(CH₂)ₜCONHR₄,
-CF₂(CH₂)ₜCH₂OR₄ ou -CF₂(CH₂)ᵥCH=CH₂, t étant 2, 3 ou 4 et v étant 1, 2 ou 3,
R₄ est H ou alkyle C₁₋₆,
A est phényle ou cyclohexyle,
B est (CH)₄ ou (CH₂)₄ qui, lorsqu'il est combiné aux deux atomes de carbone auxquels il est lié, forme une entité hydrocarbonée cyclique en C₆.

2. Composé selon la revendication 1 dans lequel l'entité R₃ est choisie dans le groupe consistant en -CF₂(CH₂)₂CH₃, -CF₂(CH₂)₂CO₂Et, -CF₂(CH₂)₂CONHCH₃ et -CF₂(CH₂)₄CONHCH₃.

3. Composé selon la revendication 2 ayant la structure de la formule IA où A est phényle, n est un, R₁ est H et R₂ est alkyle en C₁₋₇.

4. Composé selon la revendication 3 dans lequel R₂ est méthyle.

5. Composé selon la revendication 2 ayant la structure de la formule IA où A est cyclohexyle, R₁ est H et R₂ est alkyle en C₁₋₇.

6. Composé selon la revendication 5 dans lequel R₂ est méthyle et n est un.

7. Composé selon la revendication 2 ayant la structure de la formule IB où B est (CH₂)₄, chaque q est zéro, m est deux et n est zero.

8. Composé selon la revendication 2 ayant la structure de la formule IB où B est (CH₂)₄, chaque q est zéro, m est un et n est zéro ou un.

9. Composé selon la revendication 2 ayant la structure de la formule IB où B est (CH₂)₄, chaque q est un, m est un ou deux et n est zéro ou un.

10. Composé selon l'une quelconque des revendications 4, 7, 8 ou 9 dans lequel R₃ est -CF₂(CH₂)₂CH₃.

11. Procédé de préparation d'un composé répondant aux formules 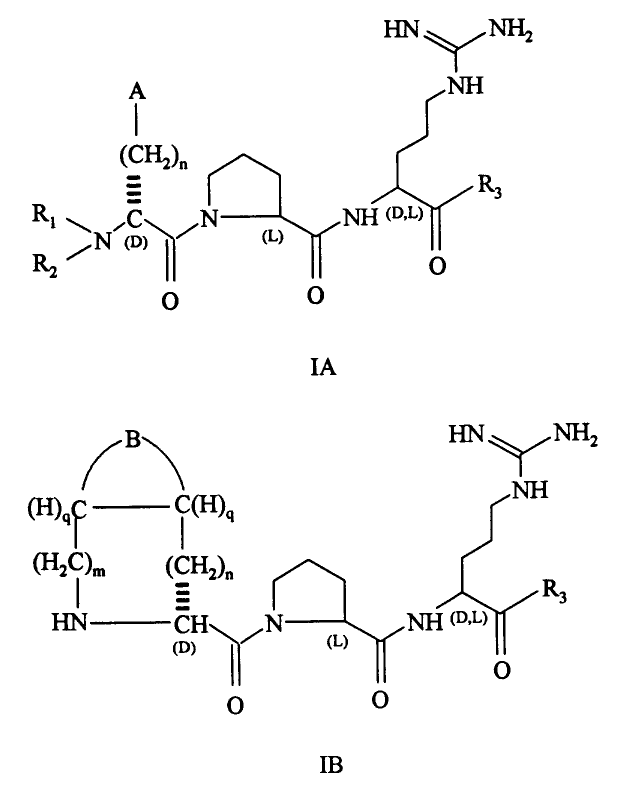 de ses isomères ou mélanges, de ses hydrates ou de ses sels pharmaceutiquement acceptables, où
m est zéro, un ou deux, n est zéro ou un, à condition que la somme de m et n soit inférieure à trois et supérieure à zéro, q est zéro ou un, à condition que la somme des deux q soit zéro ou 2,
R₁ est H ou alkyle en C₁₋₇,
R₂ est H ou alkyle en C₁₋₇, ou bien R₁ et R₂ forment avec l'atome d'azote auquel ils sont liés un hétérocycle à 5 ou 6 chaînons,
R₃ est -CF₂(CH₂)ₜCH₃, -CF₂(CH₂)ₜCOOR₄, -CF₂(CH₂)ₜCONHR₄,
-CF₂(CH₂)ₜCH₂OR₄ ou -CF₂(CH₂)ᵥCH=CH₂, t étant 2, 3 ou 4 et v étant 1, 2 ou 3,
R₄ est H ou alkyle en C₁₋₆,
A est phényle ou cyclohexyle,
B est (CH)₄ ou (CH₂)₄ qui, lorsqu'il est combiné aux deux atomes de carbone auxquels il est lié, forme une entité hydrocarbonée cyclique en C₆,
qui comprend l'oxydation d'un composé répondant aux formules 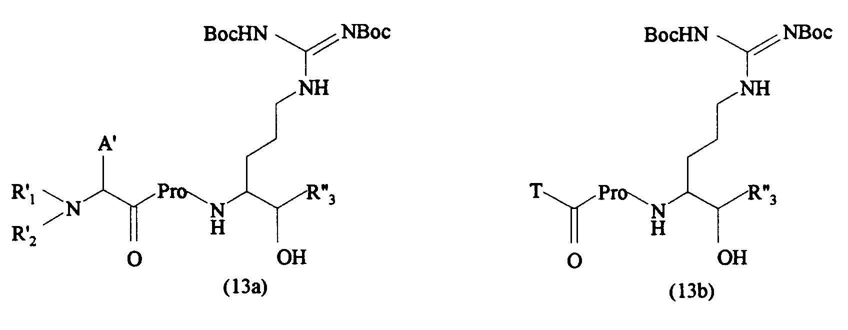 où
A' est 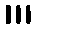 (CH₂)ₙA,
n est zéro ou un,
R'₁ est un groupe N-protecteur,
R'₂ est H ou alkyle en C₁₋₇,
R''₃ est -CF₂(CH₂)ₜCONHR₄, -CF₂(CH₂)ₜCH₂OR₄,
-CF₂(CH₂)ᵥCH=CH₂ ou -CF₂(CH₂)ₜCOOR₄,
T est une entité analogue à TIC,
t, v et R₄ étant tels que définis ci-dessus,
puis le retrait de tout groupe protecteur et (a) éventuellement la réduction de toute entité oléfinique de l'entité R₃, (b) éventuellement la N-alkylation ou la N-dialkylation de tout composé portant un groupe amino libre au niveau de l'entité P₃, et (c) éventuellement la conversion des composés ainsi produits en leurs sels pharmaceutiquement acceptables.

12. Utilisation des composés de formule IA ou IB tels que définis dans la revendication 1 pour la préparation d'un médicament.

13. Utilisation des composés de formule IA ou IB tels que définis dans le revendication 1 pour la préparation de compositions pharmaceutiques pour l'inhibition des états thrombotiques.

14. Utilisation des composés de formule IA ou IB tels que définis dans la revendication 1 pour la préparation de compositions pharmaceutiques pour le traitement de la thrombophlébite et de la thrombose coronarienne.

15. Utilisation des composés de formule IA ou IB tels que définis dans la revendication 1 pour la préparation de compositions pharmaceutiques pour l'inhibition de la tryptase pulmonaire.

16. Utilisation des composés de formule IA ou IB tels que définis dans la revendication 1 pour la préparation de compositions pharmaceutiques pour le traitement de l'asthme.

17. Composé selon la revendication 1, le composé étant le L-prolinamide, N-méthyl-D-phényl-alanyl-N-[1-(3-[(aminoiminomethyl)amino]propyl]-3,3-difluoro-2-oxohexyl]-dichlorhydrate, hydraté.
